# EUROPEAN PATENT APPLICATION

(11) **EP 4 830 633 A1**
(43) Date of publication of application: **28.10.2026**
(21) Application number: 24907628.2
(22) Date of filing: 05.06.2024
(51) Int. Cl.: A61B 6/00, A61B 6/58, A61B 90/00

(54) **MOBILE MEDICAL IMAGING DEVICE HAVING FOLDABLE ARM AND METHOD FOR OPERATING MEDICAL IMAGING DEVICE**

(30) Priority: 19.12.2023 KR 20230186305
(71) Applicant: DRTECH CORP, Seongnam-Si, Gyeonggi-do 13216 (KR)
(72) Inventor: SHIN, Choul Woo, Seongnam-si Gyeonggi-do 13476 (KR); LIM, Sung Hoon, Yongin-si Gyeonggi-do 16908 (KR); SHIN, Duck Hyun, Incheon 21321 (KR); KIM, Kun Hwan, Hwaseong-si Gyeonggi-do 18443 (KR)
(74) Representative: Jung, Minkyu
(86) International application number: PCT/KR2024/007701
(87) International publication number: WO 2025/135337

(57) **Abstract**

A medical imaging device of the present disclosure includes a main body that is able to travel, a first arm coupled to the main body by a first joint part, a second arm coupled to the first arm by a second joint part including a smart actuator, and a controller for controlling a joint part including at least one of the first joint part and the second joint part.

## Description

### [Technical Field]

The present disclosure relates to a mobile medical imaging device having a foldable arm and an operating method of the medical imaging device. More specifically, in the medical imaging device of the present disclosure, due to the foldable arm with a high degree of freedom, a source assembly can be conveniently placed at an imaging position.

### [Background Art]

Aligning a medical imaging device and a detector is an important step for obtaining a high-quality image and minimizing exposure to radiation for both a patient and a medical service provider. The following steps may be performed to align a medical imaging device and a detector.

First, the position of a source assembly may be determined. The source assembly for generating radiation should be located at a fixed distance from a region of interest of a patient. The distance varies according to the type of medical imaging device and a region being imaged but is usually about 1 to 2 meters. Next, a radiation beam may be aligned. A radiation beam should be aligned to be perpendicular to a detector and pass through the region of interest of the patient. The alignment may be performed by adjusting the position of the source assembly or forming the radiation beam using a collimator.

Next, the position of the detector may be designated. The detector should be located at the opposite side of the patient relative to the source assembly and should be aligned with the radiation beam. In addition, the detector may be disposed as close as possible to the patient to minimize scattered radiation and improve image quality. Lastly, an alignment checking step may be performed. When the source assembly and the detector are at their correct positions, a test image may be captured, and the alignment may be checked. Whether the region of interest is at the center of the image and the image quality is sufficient for diagnosis may be checked.

Since the source assembly is on the heavy side, it is difficult for a user to manually place the source assembly close to the patient or align the source assembly with the detector in some cases. In particular, in the case of the mobile medical imaging device, since it is necessary to not only move the source assembly but also place the main body near the patient, effort made by the user or patient may further increase compared to a stationary medical imaging device. Accordingly, research on a mobile medical imaging device using an arm with a high degree of freedom to move a source assembly is underway.

### [Related Art Document]

### [Patent Document]

(Patent Document 1) Korean Patent Registration No. 10-1616670 (April 28, 2016)

### [Disclosure]

### [Technical Problem]

The present disclosure discloses a mobile medical imaging device having an arm with a high degree of freedom of movement.

### [Technical Solution]

A medical imaging device according to the present disclosure includes a main body that is able to travel, a first arm coupled to the main body by a first joint part, a second arm coupled to the first arm by a second joint part including a smart actuator, and a controller for controlling the second joint part.

The controller of the medical imaging device according to the present disclosure may control the second joint part to rotate the second arm relative to the first arm, based on at least one of a torque applied to the second joint part and a user input.

The controller of the medical imaging device according to the present disclosure may measure a first torque applied to the second joint part, may determine whether the first torque is higher than or equal to a predetermined threshold sensitivity torque of the second joint part, and when the first torque is higher than or equal to the threshold sensitivity torque of the second joint part, may control the second joint part to rotate the second arm relative to the first arm, and the threshold sensitivity torque of the second joint part may be changeable.

The controller of the medical imaging device according to the present disclosure may control the second joint part so that a predetermined angle is formed between the first arm and the second arm based on a user input on a button related to joint movement.

The medical imaging device according to the present disclosure may further include a source assembly that is coupled to the other end of the second arm and includes a second transceiver and a detector that generates a medical image by receiving radiation radiated from the source assembly and includes a first transceiver transmitting and receiving a signal to and from the second transceiver, and based on the first transceiver and the second transceiver, the controller may control the second joint part so that a radiation irradiation direction of the source assembly becomes perpendicular to a radiation reception surface of the detector.

The controller of the medical imaging device according to the present disclosure may obtain a second torque caused by an external force while the second arm moves relative to the first arm due to driving of the second joint part, may determine whether the second torque is higher than or equal to a predetermined threshold impact torque, and when the second torque is higher than or equal to the predetermined threshold impact torque, may stop the driving of the second joint part.

The second arm of the medical imaging device according to the present disclosure may include: a second-first arm that has one end coupled to the second joint part; a second-second arm that has at least one portion inserted into a space formed inside the second-first arm and is able to move along the second-first arm; and a telescopic arm driving part that is coupled to an inner portion of the second-first arm and provides a driving force for movement of the second-second arm relative to the second-first arm.

The second joint part of the medical imaging device according to the present disclosure may include a first smart actuator coupled to one side of at least one of the first arm and the second arm and a second smart actuator coupled to the other side of at least one of the first arm and the second arm, and the first smart actuator and the second smart actuator may provide a driving force to an axis of rotation of the second arm relative to the first arm.

A medical imaging device according to the present disclosure includes a main body that is able to travel, a first arm coupled to the main body by a first joint part, a second arm coupled to the first arm by a second joint part and stretchable/contractible by a telescopic arm driving part, and a controller for controlling the second joint part.

The second arm of the medical imaging device according to the present disclosure may include a second-first arm that has one end coupled to the second joint part, a second-second arm that has at least one portion inserted into a space formed inside the second-first arm and is able to move along the second-first arm, and a telescopic arm driving part that is coupled to an inner portion of the second-first arm and controls a driving force for movement of the second-second arm relative to the second-first arm.

The controller of the medical imaging device according to the present disclosure may control the second-second arm to move relative to the second-first arm based on one of a user input on a button related to a telescopic function and a force applied to the second-second arm by the user.

The controller of the medical imaging device according to the present disclosure may obtain an outer force caused by an external force while the second-second arm moves relative to the second-first arm due to the telescopic arm driving part, may determine whether the outer force is greater than or equal to a predetermined threshold impact force, and when the outer force is greater than or equal to the predetermined threshold impact force, may stop the driving of the telescopic arm driving part.

In addition, a program for implementing an operation method of the medical imaging device described above may be recorded in a computer-readable recording medium.

### [Advantageous Effects]

A mobile medical imaging device of the present disclosure allows a user to easily place a source assembly facing the user using an arm with a high degree of freedom. In addition, since the arm supports the weight of the source assembly, the user can move the source assembly without much effort.

In addition, by providing a means for promptly aligning the source assembly and a detector of the mobile medical imaging device of the present disclosure, user convenience can be enhanced, and medical image quality can be improved.

Advantageous effects that can be obtained by the present disclosure are not limited to those mentioned above, and other unmentioned advantageous effects should be clearly understood by those of ordinary skill in the art to which the present disclosure pertains from the following description.

### [Description of Drawings]

FIG. 1 is a view showing a mobile medical imaging device according to one embodiment of the present disclosure.
FIG. 2 is a view showing a process of using the medical imaging device according to one embodiment of the present disclosure.
FIG. 3 is a view showing a block diagram of various components that may be included in the medical imaging device according to one embodiment of the present disclosure.
FIG. 4 shows a source arm of the medical imaging device according to one embodiment of the present disclosure.
FIG. 5 is a view for describing a second joint part according to one embodiment of the present disclosure.
FIG. 6 is a flowchart for describing an operation of the medical imaging device according to one embodiment of the present disclosure.
FIG. 7 is a flowchart showing an operation of the medical imaging device according to one embodiment of the present disclosure.
FIG. 8 is a view for describing angular acceleration of a second arm of the present disclosure.
FIG. 9 is a view for describing a degree of freedom of an arm of the medical imaging device according to one embodiment of the present disclosure.
FIG. 10 is a view for describing a component for moving the arm of the medical imaging device according to one embodiment of the present disclosure.
FIG. 11 shows a plan view of the medical imaging device according to one embodiment of the present disclosure.
FIG. 12 is a view for describing the second joint part according to one embodiment of the present disclosure.
FIG. 13 is a view for describing a movement brake of a main body according to one embodiment of the present disclosure.

### [Modes of the Invention]

Advantages and features of the disclosed embodiments and methods for achieving them will become clear by referring to the embodiments described below in conjunction with the accompanying drawings. However, the present disclosure is not limited to the embodiments disclosed below and may be implemented in various different forms, and these embodiments are provided only to make the disclosure complete and fully inform those of ordinary skill in the art to which the present disclosure pertains of the scope of the invention.

Terms used in this specification will be briefly described, and the disclosed embodiments will be described in detail.

General terms that are currently widely used have been selected as terms used in this specification in consideration of functions in the present disclosure, but the terms may vary depending on the intentions of engineers working in the related fields, precedents, the emergence of new technologies, or the like. Additionally, there may be terms deliberately selected by the applicants, and in such a case, their meanings will be described in detail in the corresponding part of the description of the invention. Accordingly, the terms used in this disclosure should be defined based on the meanings of the terms and the overall content of the present disclosure, rather than simply the names of the terms.

In this specification, a singular expression includes a plural expression unless the context clearly indicates singularity. Also, a plural expression includes a singular expression unless the context clearly indicates plurality.

Throughout the specification, when a certain part is described as "including" a certain component, this indicates that the certain part may further include other components instead of excluding other components unless the context clearly indicates otherwise.

Also, the term "-er/-or" or "part" used in this specification refers to a software or hardware component, and an "-er/-or" or "part" performs certain roles. However, the meaning of "-er/-or" or "part" is not limited to software or hardware. An "-er/-or" or "part" may be configured to be present in addressable storage media or configured to replay one or more processors. Therefore, as one example, an "-er/-or" or "part" may include components such as software components, object-oriented software components, class components, and task components, processes, functions, attributes, procedures, subroutines, program code segments, drivers, firmware, micro codes, circuits, data, databases, data structures, tables, arrays, or variables. The components and functions provided in "-ers/-ors" or "parts" may be combined into smaller numbers of components and "-ers/-ors" or "parts" or may be further separated into larger numbers of components and "-ers/-ors" or "parts."

According to one embodiment of the present disclosure, an "-er/-or" or "part" may be implemented using a processor and a memory. The term "processor" should be interpreted in a wide sense to include a universal processor, a central processing unit (CPU), a microprocessor, a digital signal processor (DSP), a controller, a microcontroller, a state machine, and the like. In some environments, "processor" may also refer to an application-specific integrated circuit (ASIC), a programmable logic device (PLD), a field programmable gate array (FPGA), or the like. For example, the term "processor" may also refer to a combination of processing devices such as a combination of a DSP and a microprocessor, a combination of a plurality of microprocessors, a combination of one or more microprocessors combined with a DSP core, or a combination of other arbitrary configurations.

The term "memory" should be interpreted in a wide sense to include an arbitrary electronic component that can store electronic information. The term "memory" may also refer to various types of processor-readable media such as a random access memory (RAM), a read-only memory (ROM), a nonvolatile random access memory (NVRAM), a programmable read-only memory (PROM), an erasable-programmable read-only memory (EPROM), an electrically erasable PROM (EEPROM), a flash memory, a magnetic or optical data storage device, and registers. When a processor is able to read information from a memory and/or record information in the memory, the memory is referred to as being in an electronic communication state with the processor. A memory integrated in a processor is in an electronic communication state with the processor.

Hereinafter, embodiments will be described in detail with reference to the accompanying drawings to allow those of ordinary skill in the art to which the present disclosure pertains to easily carry out the embodiments. Also, parts unrelated to the description are omitted from the drawings to clearly describe the present disclosure.

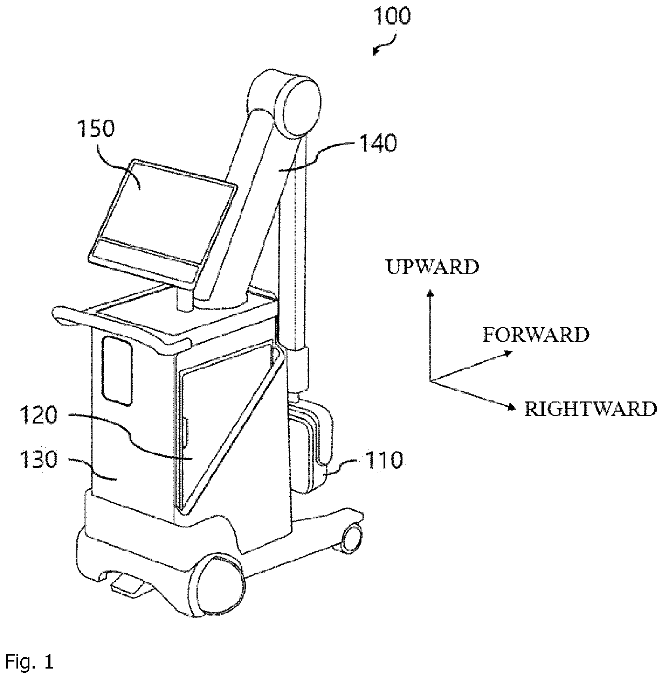

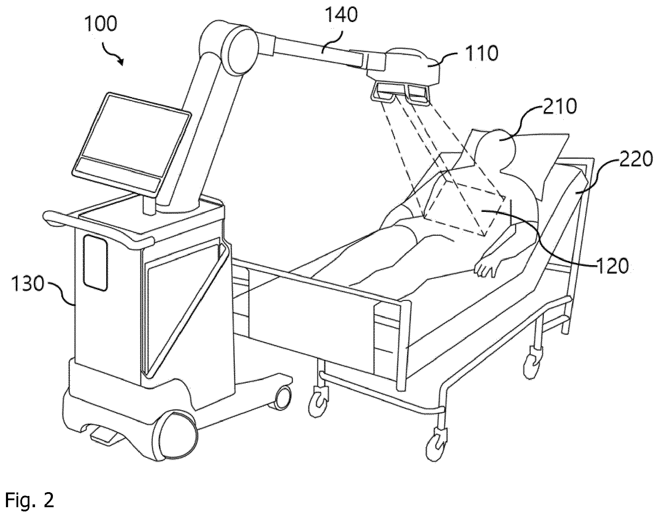

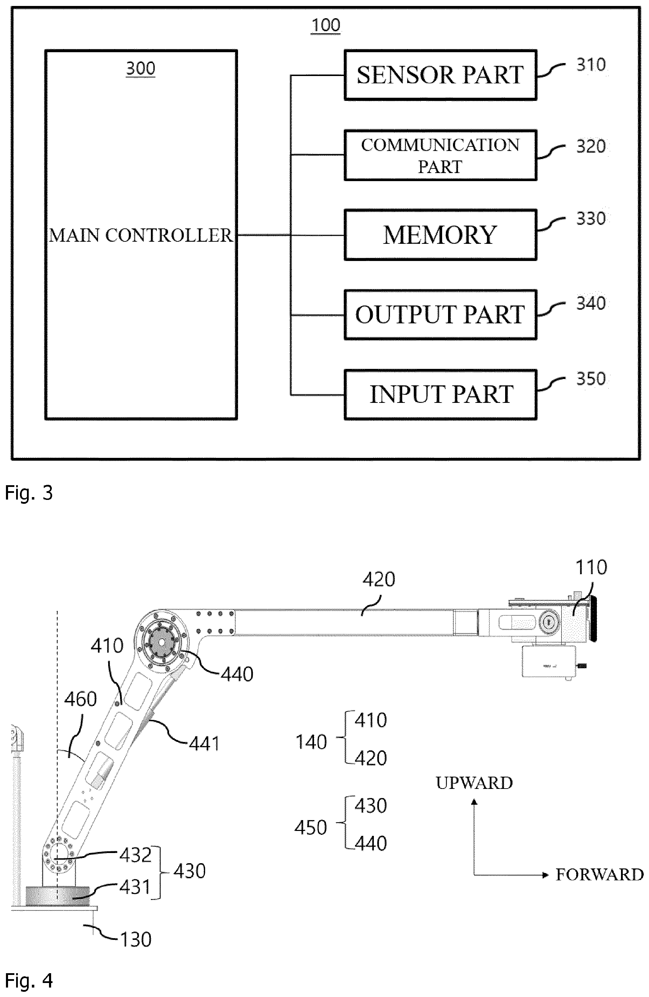

FIG. 1 is a view showing a mobile medical imaging device according to one embodiment of the present disclosure. In addition, FIG. 2 is a view showing a process of using the medical imaging device according to one embodiment of the present disclosure. In addition, FIG. 3 is a view showing a block diagram of various components that may be included in the medical imaging device according to one embodiment of the present disclosure.

Referring to FIG. 1, a mobile medical imaging device 100 of the present disclosure may include wheels and be able to move. A medical imaging device according to one embodiment may be a device that can capture an image of and/or examine a structure inside a subject (or object) based on radiation including x-rays. For example, a medical imaging device irradiates the human body with x-rays so that the x-rays penetrate the human body, scans the penetrating x-rays, and obtains an image of the inside of the human body.

Referring to FIGS. 1 to 3, the medical imaging device 100 may include a source assembly 110, a detector 120, and a main body 130. In addition, the main body 130 of the medical imaging device 100 may include a high-voltage generator (not illustrated in the drawings), a sensor part 310, a communication part 320, a memory 330, an output part 340, an input part 350, and/or a controller 300.

Referring to FIG. 2, the main body 130 may be able to travel. The main body 130 may include wheels. The wheels may include at least one of castor wheels, electric wheels, and omni wheels. The main body 130 may move due to a force exerted by a user or may automatically move due to a wheel actuator.

A user may move the medical imaging device 100 to the vicinity of a hospital bed 220. The user may place the detector 120 behind a subject 210. Accordingly, the radiation radiated from the source assembly 110 may pass through the subject 210 and reach the detector 120. The detector 120 may detect the radiation passing through the subject 210 and may convert the radiation into an electrical signal. In addition, the detector 120 may obtain a radiation image based on the electrical signal.

The medical imaging device 100 may include a source arm 140. The source assembly 110 may be connected to the main body 130 through the source arm 140. The source assembly 110 may include an x-ray source and a collimator. The x-ray source may be a component that radiates radiation. The x-ray source may be rotatable about an axis parallel to the longitudinal direction of the source arm 140.

In addition, a radiation irradiation range may be determined by the collimator. The collimator may rotate relative to the x-ray source about an axis parallel to a radiation irradiation direction.

Referring to FIGS. 1 to 3, the high-voltage generator according to one embodiment may generate a high voltage for x-ray generation and may apply the high voltage to the x-ray source included in the source assembly. The high-voltage generator may be included in the main body 130, but is not limited thereto, and may also be included in the source assembly 110.

The source assembly 110 according to one embodiment may include the x-ray source that receives the high voltage generated by the high-voltage generator and generates x-rays. The x-ray source may include an x-ray tube, and the x-ray tube may be implemented as a diode vacuum tube consisting of a cathode and an anode. In addition, the source assembly may include the collimator that guides a path of the x-rays radiated from the x-ray source and adjusts an x-ray irradiation region.

The detector according to one embodiment detects the x-rays irradiated from the source assembly and passing through an object. The detector may be a digital detector. The detector may be implemented using at least one of a thin film transistor (TFT), a charge coupled device (CCD), a complementary metal-oxide semiconductor (CMOS), computed radiography (CR), and a film. The detector may be included in the medical imaging device 100 or may be a separate device that can be connected to and separated from the medical imaging device 100.

The medical imaging device 100 may include the controller 300. In the present disclosure, the controller 300 may mean at least one of a main controller included in the main body 130 and a detector controller included in the detector. In the present disclosure, a controller included in the main body is referred to as "main controller," and for a controller included in another device, a device in which the controller is included is clearly indicated. For example, a detector controller is a controller included in a mobile detector and may be a controller different from the main controller 300. The main controller 300 and the detector controller are included in different devices but may be similar in that each may include at least one of a processor and a memory. At least some of the operations of the main controller may be performed by the detector controller. In addition, at least some of the operations of the detector controller may be performed by the main controller. Accordingly, in the present disclosure, at least one operation described as an operation of the main controller may be understood as being performed by the detector controller, and at least one operation described as an operation of the detector controller may be understood as being performed by the main controller.

The main controller 300 (or controller 300) may control an operation of the medical imaging device 100. For example, the medical imaging device 100 may include the main controller 300 for controlling an operation of the source assembly 110 or a wheel actuator allowing the main body 130 to travel. The main controller 300 may include one processor or may include a plurality of processors. The main controller 300 may be included in the main body 130. When the main controller 300 includes a plurality of processors, at least some of the plurality of processors may be provided at positions physically apart from the main body 130. In addition, the medical imaging device 100 is not limited thereto and may be implemented in various other ways.

According to one embodiment of the present disclosure, the main controller 300 may control an operation of the medical imaging device 100. For example, the medical imaging device 100 may include a plurality of actuators, and the medical imaging device 100 may control an operation of the medical imaging device 100 by controlling operations of the plurality of actuators. For example, the main controller 300 may control a source assembly driver for moving the source assembly 110. In addition, the main controller 300 may obtain an x-ray image by controlling the source assembly 110 to radiate x-rays and the detector 120 to receive the x-rays passing through an object.

According to one embodiment of the present disclosure, the main controller 300 may generate a medical image. For example, the main controller 300 may generate a medical image by scanning the detector irradiated with x-rays.

The medical imaging device 100 may include the sensor part 310. The sensor part 310 may obtain various information using at least one sensor. The sensor part 310 may be provided as a sensor using a means of measurement such as pressure, potential, and an optical means. For example, the sensor part 310 may include at least one of a distance measurement sensor and an encoder. In addition, the sensor part may include a pressure sensor, an infrared sensor, a light emitting diode (LED) sensor, a touch sensor, or the like. However, the sensor part is not limited thereto. The sensor part may be included in at least one of the main body, the source assembly, the detector, a source assembly arm, and a detector arm.

In addition, the medical imaging device 100 may include the communication part 320. The communication part 320 may be a component allowing the medical imaging device 100 to communicate with an internal module or an external device in a wired or wireless manner. The external device may be an external server or a user terminal. The user terminal may be a personal computer (PC), a smartphone, a tablet, or a wearable device. The communication part 320 may include a wired/wireless communication module for network connection. As a wireless communication technology, for example, wireless LAN (WLAN) (Wi-Fi), wireless broadband (WiBro), World Interoperability for Microwave Access (WIMAX), High Speed Downlink Packet Access (HSDPA), etc., may be used. As a wired communication technology, for example, Digital Subscriber Line (xDSL), Fiber To The Home (FTTH), Power Line Communication (PLC), etc., may be used. In addition, a network connection part may include a short-range communication module and may transmit and receive data to and from an arbitrary device/terminal located at a short distance. For example, as a short-range communication technology, Bluetooth, Radio Frequency Identification (RFID), Infrared Data Association (IrDA), Ultra-Wideband (UWB), ZigBee, etc., may be used, but the short-range communication technology is not limited thereto.

The medical imaging device 100 may include the memory 330. The main controller 300 may execute instructions stored in the memory. The memory 330 may be included in the main controller 300 or may be present outside the main controller 300. The memory 330 may store various information related to the medical imaging device 100. For example, the memory 330 may include information related to an operation method of the source assembly 110 or may include captured images and user authentication information, but is not limited thereto.

The memory 330 may be implemented using a nonvolatile storage medium that can continuously store arbitrary data. Examples of the memory 330 may include not only a disk, an optical disk, and a magneto-optical storage device, but also a flash memory and/or a storage device based on a battery-backup memory, but the memory 330 is not limited thereto. The memory 330 may be a volatile storage device that is a main storage device directly accessed by a processor and in which stored information is instantly erased when power is turned off, such as a random access memory (RAM) including a dynamic random access memory (DRAM) and a static random access memory (SRAM), but the memory 330 is not limited thereto. The memory 330 may be operated by the main controller 300. In addition, the main controller 300 may execute instructions included in the memory 330.

In addition, the medical imaging device 100 may further include a manipulation part providing an interface for manipulation of the medical imaging device 100. The manipulation part may include the output part 340 and the input part 350.

The output part 340 may show imaging-related information such as irradiation of x-rays under control of the main controller 300 or may output sound and an image allowing a state of the main body to be checked. The output part 340 may include a speaker or a display. The output part 340 may include at least one of a main display 150 included in the main body 130 and a sub display included in the source assembly 110. The output part 340 may output a medical image generated by the main controller 300. The output part 340 may output information necessary for a user to manipulate the medical imaging device 100, such as a user interface (UI), user information, or object information. Examples of the output part 340 may include a speaker, a printer, a cathode ray tube (CRT) display, a liquid crystal display (LCD), a plasma display panel (PDP), an organic light emitting diode (OLED) display, a field emission display (FED), a light emitting diode (LED) display, a vacuum fluorescent display (VFD), a digital light processing (DLP) display, a flat panel display (FPD), a 3D display, a transparent display, etc., and may include various other output devices within the scope apparent to those skilled in the art.

The medical imaging device 100 may be connected to a work station via a wire or wirelessly. The work station may be present in a space physically separated from the medical imaging device 100.

The work station may include a storage server. The storage server may store a medical image, information on an object, information on a user (medical service provider), etc. The work station may include a review device. The review device may receive a medical image from the storage server and diagnose the medical image based on a user command. The work station and the medical imaging device 100 may send, store, process, and output data according to the Digital Imaging and Communications in Medicine (DICOM) standards. In addition, the work station may include a Picture Archiving and Communication System (PACS).

The work station may include an output part, an input part, and a controller. The output part and the input part provide an interface for manipulation of the work station and the medical imaging device 100 to the user. The controller of the work station may control the work station and the medical imaging device 100.

The medical imaging device 100 may be controlled through the work station or may be controlled by the main controller 300 included in the medical imaging device 100. Accordingly, the user may control the medical imaging device 100 through the work station or may control the medical imaging device 100 through the manipulation part and the main controller 300 included in the medical imaging device 100. In other words, the user may remotely control the medical imaging device 100 through the work station or may directly control the medical imaging device 100.

The controller of the work station and the main controller 300 of the medical imaging device 100 may be separate controllers, but the present disclosure is not limited thereto. The controller of the work station and the main controller 300 of the medical imaging device 100 may be implemented as one integrated controller, and the integrated controller may be included in only one of the work station and the medical imaging device 100. In the following description, the main controller 300 may indicate the controller of the work station and/or the controller of the medical imaging device 100.

The output part and the input part of the work station and the output part 340 and the input part 350 of the medical imaging device 100 may each provide an interface for manipulation of the medical imaging device 100 to the user. The work station and the medical imaging device 100 may each include an output part and an input part, but the present disclosure is not limited thereto. An output part or an input part may be implemented in only one of the work station and the medical imaging device 100.

In the following description, the input part 350 indicates the input part of the work station and/or the input part of the medical imaging device 100, and the output part 340 indicates the output part of the work station and/or the output part of the medical imaging device 100.

The input part 350 may receive commands for manipulating the medical imaging device 100 and various information related to x-ray imaging from the user. The main controller 300 may control or manipulate the medical imaging device 100 based on the information input through the input part 350. The input part 350 may include a joystick, a keyboard, a mouse, a touchscreen, an imaging button, an unlocking button, a voice recognition device, a fingerprint recognition device, an iris recognition device, a human body motion recognition device, etc., and may include other input devices apparent to those skilled in the art.

The human body motion recognition device included in the input part 350 may be implemented using at least one camera. For example, the human body motion recognition device may be implemented using a 3D camera or a depth sensor included in the source assembly 110. The main controller 300 may control an operation of the medical imaging device 100 based on the human body motion recognition device.

User convenience can be improved because the medical imaging device 100 is controlled based on gestures. For example, because a user can control the medical imaging device 100 at an arbitrary position without going back to the main body 130, movements of the user when capturing a medical image can be reduced. In addition, because there is no need for the user to be close to the medical imaging device 100 to input a gesture, the user's exposure to radiation can be reduced, and the user's safety can be ensured.

The user may input a command for x-ray irradiation through the input part 350, and a switch for input of such a command may be provided on the input part 350. The switch may be provided so that an irradiation command for x-ray irradiation is input when the switch is pressed at least once.

For example, the switch may have a structure in which, when a user presses the switch, a preparation command instructing that preheating for x-ray irradiation be performed is input, and in that state, when the user presses the switch deeper, an irradiation command for actual x-ray irradiation is input. When the user manipulates the switch in this way, the main controller 300 generates a signal corresponding to a command input through manipulation of the switch, that is, a preparation signal, and transmits the preparation signal to the high-voltage generator generating a high voltage for x-ray generation.

The high-voltage generator receives the preparation signal transmitted from the main controller 300, starts preheating, and when preheating is complete, transmits a preparation complete signal to the main controller 300. In addition, preparation for x-ray detection is also necessary for the detector to detect x-rays, and the main controller 300 transmits the preparation signal to the detector so that, while preheating is performed by the high-voltage generator, the detector prepares for detection of x-rays passing through an object. Upon receiving the preparation signal, the detector prepares for x-ray detection, and when preparation for detection is complete, the detector transmits a preparation-for-detection complete signal to the main controller 300.

When preheating of the high-voltage generator is complete, and preparation for x-ray detection of the detector is complete, the main controller 300 transmits an irradiation signal to the high-voltage generator, the high-voltage generator generates a high voltage and applies the high voltage to the x-ray source, and the x-ray source irradiates x-rays.

When transmitting the irradiation signal, the controller 300 may transmit a sound or light output signal to the output part 340 and may allow predetermined sound or light to be output from the output part 340 so that an object can become aware of x-ray irradiation. In addition, sound or light indicating imaging-related information other than x-ray irradiation may be output from the output part 340. The output part 340 may be included in the manipulation part but is not limited thereto, and the output part 340 or a portion of the output part 340 may be located at a different point from a point where the manipulation part is located. For example, the output part 340 or a portion of the output part 340 may be located on a wall of an imaging room where x-ray imaging is performed for an object.

The controller 300 controls positions of an x-ray irradiation part and the detector, an imaging timing, an imaging condition, and the like according to imaging conditions set by the user.

Specifically, the main controller 300 controls the high-voltage generator and the detector and controls an x-ray irradiation timing, an x-ray intensity, an x-ray irradiation region, etc., according to a command input through the input part 350. In addition, the main controller 300 adjusts the position of the detector and controls an operation timing of the detector according to predetermined imaging conditions.

In addition, the main controller 300 generates a medical image of an object using image data received through the detector. Specifically, the main controller 300 may generate a medical image of an object by receiving image data from the detector, removing noise from the image data, and adjusting a dynamic range and interleaving.

The work station may further include a communication part (not illustrated) that can be connected to a server, a medical device, a portable terminal, etc., through a network. The work station may be one of external devices.

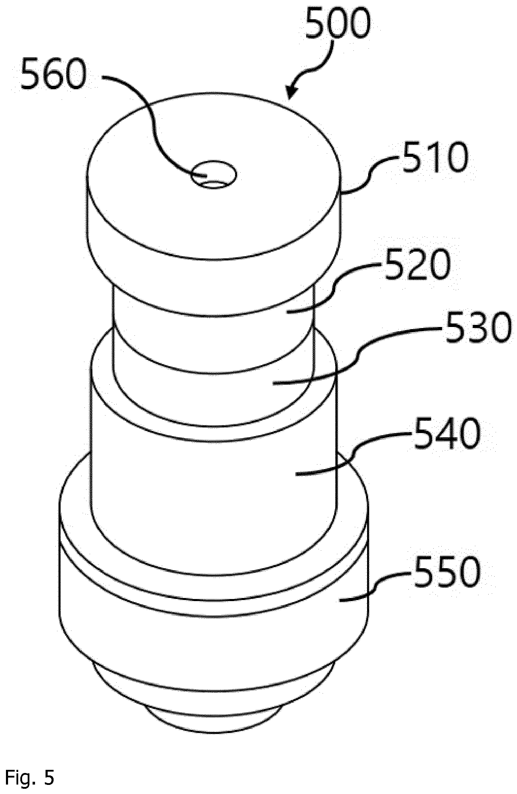

The source assembly 110 and the detector 120 will be described in detail below with reference to FIGS. 4 and 5.

FIG. 4 shows a source arm of the medical imaging device according to one embodiment of the present disclosure.

The source arm 140 may include a first arm 410, a second arm 420, a first joint part 430, and a second joint part 440.

The first arm 410 may be coupled to the main body 130 by the first joint part 430. The medical imaging device 100 of the present disclosure may include a joint part 450. The joint part 450 may include at least one of the first joint part 430 and the second joint part 440. In addition, the first joint part 430 may include a first-first joint part 431 and a first-second joint part 432.

The first-first joint part 431 may rotate the first arm 410 about an axis parallel to the upward direction. That is, the first-first joint part 431 may rotate the first arm 410 about an axis perpendicular to the ground. The first-first joint part 431 may include a friction brake. Since the friction brake may operate at all times, the first arm 410 may not rotate about the axis parallel to the upward direction. For example, since a first friction plate fixed to the main body 130 and a second friction plate fixed to the first arm 410 are in contact with each other, the first arm 410 may not rotate about the axis parallel to the upward direction. However, when an input on a brake release button that is related to the first-first joint part 431 is received from a user, the main controller 300 may release the friction brake and allow the first arm 410 to rotate about the axis parallel to the upward direction. More specifically, the first friction plate and the second friction plate receiving the input on the brake release button from the user may be spaced from each other and allow the first arm 410 to rotate about the axis parallel to the upward direction. A force for the first friction plate and the second friction plate to be spaced from each other may be provided by a magnetic force or a driving force of a motor.

A maximum angle of rotation of the first arm 410 about the axis parallel to the upward direction may be less than or equal to 20° rightward and less than or equal to 20° leftward. More specifically, the maximum angle of rotation of the first arm 410 about the axis parallel to the upward direction may be less than or equal to 15° rightward and less than or equal to 15° leftward. By limiting the angle of rotation of the first arm 410 about the axis parallel to the upward direction in this way, balance of the main body 130 can always be maintained. The source assembly 110, which is heavy, may be coupled to the source arm 140, and when the first arm 410 excessively rotates about the axis parallel to the upward direction, the main body 130 may lose balance and fall sideways. However, by limiting the angle of rotation of the source arm 140, the medical imaging device 100 of the present disclosure can prevent the situation in which the main body 130 loses balance and falls.

The configuration in which the first-first joint part 431 rotates relative to the main body 130 about the axis perpendicular to the ground has been described above, but the present disclosure is not limited thereto. The first-first joint part 431 may not be rotatable relative to the main body 130 about the axis perpendicular to the ground. That is, the first-first joint part 431 may be fixed to not be movable relative to the main body 130. The user may rotate the main body 130 itself when rotation of the source arm 140 about the axis perpendicular to the ground is necessary.

The first-second joint part 432 may be a component for coupling the main body 130 and the first arm 410. The first-second joint part 432 may be a component for coupling the first-first joint part 431 and the first arm 410. The first-second joint part may have a fixed structure. That is, the first-second joint part may not be rotatable. The first-second joint part may not be rotatable about the axis parallel to the ground. The first-second joint part may include a plurality of fixing screws. The plurality of fixing screws may be components for coupling the first-first joint part 431 and the first arm 410. The first-second joint part 432 may have to withstand high torque caused by the weights of the source arm 140 and the source assembly 110. By including the plurality of fixing screws, the first-second joint part 432 may withstand high torque acting on the first joint part 430.

Due to the first-second joint part 432, the first arm 410 may be fixed while tilted at a predetermined fixed angle relative to the ground. Due to the first-second joint part 432, the first arm 410 may be fixed while tilted at a predetermined fixed angle 460 relative to a line perpendicular to the ground. The predetermined fixed angle 460 may be 10° or more and 60° or less.

The predetermined fixed angle 460 may be determined according to the onsite situation. For example, the predetermined fixed angle 460 may increase on a site with a low ceiling. In addition, the predetermined fixed angle 460 may be changed based on the size of a bed placed on the site. For example, when the size of a bed placed on the site has a size of (horizontal length × vertical length × height), and the vertical length is longer than the horizontal length, the range of the angle may be determined according to the following equation.

Vertical length of bed/2 - predetermined allowed length <= length of first arm × sin(predetermined fixed angle) + length of second arm <= vertical length of bed/2 + predetermined allowed length

Here, the predetermined allowed length may have a value of 10 cm or more and 40 cm or less.

The case in which the first-second joint part 432 is fixed has been described above, but the present disclosure is not limited thereto. The first-second joint part 432 may be rotatable about the axis parallel to the ground.

More specifically, the first-second joint part 432 may rotate the first arm 410 about an axis parallel to the left-right direction. The first-second joint part 432 may include a smart actuator. The first-second joint part 432 may be a component for supporting the first arm 410. That is, the first-second joint part 432 may generate torque in the opposite direction of the torque caused by the weight of the source arm 140 and may prevent the first arm 410 from moving. The smart actuator may include a magnetic brake to generate torque in the opposite direction of the torque caused by the weight of the source arm 140 when the first-second joint part 432 is stationary. In addition, the smart actuator may include a joint motor to generate torque in the opposite direction of the torque caused by the weight of the source arm 140 when the first-second joint part 432 is moving.

The first-second joint part 432 may be a component for rotating the first arm 410 about the axis parallel to the left-right direction. The first-second joint part 432 may be a component for rotating the first arm 410 about the axis parallel to the ground. The first-second joint part 432 may rotate the first arm 410 about the axis parallel to the left-right direction or the ground by user input or control of the controller 300.

The second arm 420 may be coupled to the first arm 410 by the second joint part 440. The second arm 420 may be stretchable/contractible by a telescopic arm driving part 1030. However, the present disclosure is not limited thereto, and the second arm 420 may not be stretchable/contractible. The second arm 420 may rotate relative to the first arm 410 about the axis parallel to the left-right direction or the ground due to the second joint part 440. The second arm 420 may be a component for placing the source assembly 110 near a patient.

The second joint part 440 may include a smart actuator. The second joint part 440 may be a component for moving the second arm 420 relative to the first arm 410. The smart actuator may be a component including at least one of a joint motor 540, a harmonic drive 550, a torque sensor, and a joint encoder 520. The smart actuator will be described later. The second joint part 440 may allow the second arm 420 to move relative to the first arm 410 based on a user input or a signal of the controller 300. The second arm 420 may move relative to the first arm 410 about an axis parallel to the ground. For example, the second arm 420 may rotate relative to the first arm 410 about an axis extending in the left-right direction. An operation of the second joint part 440 will be described later.

In addition, the second joint part 440 may be a component for fixing the second arm 420 relative to the first arm 410. The second joint part 440 may receive torque due to weights of the second arm 420 and the source assembly 110. For example, the direction of the torque that the second joint part 440 receives due to the weights of the second arm 420 and the source assembly 110 may be clockwise. The smart actuator included in the second joint part 440 may provide torque for offsetting the torque received due to the weights of the second arm 420 and the source assembly 110. For example, when the second arm 420 is stationary relative to the first arm 410, a magnetic brake 530 may provide torque for offsetting the torque that the second joint part 440 receives due to the weights of the second arm 420 and the source assembly 110. The smart actuator included in the second joint part 440 may allow the second arm 420 to be fixed relative to the first arm 410. In addition, when the second arm 420 is moving relative to the first arm 410, the joint motor 540 may provide torque for offsetting the torque that the second joint part 440 receives due to the weights of the second arm 420 and the source assembly 110.

According to various embodiments of the present disclosure, the second joint part 440 may further include a gas spring 441. The gas spring 441 may provide torque for offsetting the torque that the second joint part 440 receives due to the weights of the second arm 420 and the source assembly 110. That is, the gas spring 441 and the smart actuator included in the second joint part 440 may provide torque to prevent the second arm 420 from moving relative to the first arm 410. In addition, the gas spring 441 may be a component for reducing a burden of the smart actuator. This is because, if the gas spring 441 were not present, the smart actuator would have to withstand the torque caused by the weight of the source assembly 110 alone.

FIG. 5 is a view for describing the second joint part according to one embodiment of the present disclosure.

The second joint part 440 may include a smart actuator 500. A structure of the smart actuator 500 will be described with reference to FIG. 5. The smart actuator 500 may include at least one of a motor drive 510, the joint encoder 520, the magnetic brake 530, the joint motor 540, and the harmonic drive 550. The motor drive 510, the joint encoder 520, the magnetic brake 530, the joint motor 540, and the harmonic drive 550 may be arranged along a driving shaft 560 of the smart actuator 500. Although not shown in FIG. 5, the smart actuator 500 may further include a torque sensor.

The motor drive 510 may include a control board for driving the joint motor 540. The motor drive 510 may generate a signal for driving the joint motor 540 based on a signal of the controller 300 of the main body 130. The motor drive 510 may transmit a signal of the joint encoder according to rotation of the joint motor to the controller 300 of the main body 130. In addition, the motor drive 510 may control the magnetic brake 530. The magnetic brake 530 may be in a state in which the brake operates at all times. The magnetic brake 530 may allow the brake to be released when the joint motor 540 rotates based on a signal of the motor drive 510. For example, when a user presses a brake release button, the magnetic brake 530 may be released, and the smart actuator may reach a state in which it is movable.

The joint encoder 520 may be a component for measuring at least one of an angle of rotation, a rotational velocity, and a rotational acceleration of the driving shaft of the smart actuator 500. The joint encoder 520 may be a multi-turn absolute value encoder. An angle of rotation of the joint motor may indicate a rotational position of the second arm 420 relative to the first arm 410. That is, the joint encoder 520 may measure the position of the second arm 420 relative to the first arm 410. In addition, the joint encoder 520 may measure a rotational velocity of the second arm 420 relative to the first arm 410. The rotational velocity may be information including a rotational direction and a rotational speed. The joint encoder 520 may measure a rotational acceleration of the second arm 420 relative to the first arm 410. The joint encoder 520 may also serve as a torque sensor. However, the present disclosure is not limited thereto, and the smart actuator 500 may have a separate torque sensor. The controller 300 may perform necessary control based on the angle of rotation, the rotational velocity, and the rotational acceleration measured by the joint encoder 520.

The magnetic brake 530 may be a component for fixing the driving shaft of the smart actuator 500 to prevent rotation thereof. As described above, the smart actuator 500 included in the second joint part 440 may be a component for rotating or fixing the second arm 420 relative to the first arm 410. The magnetic brake 530 may be a component for fixing the second arm 420 relative to the first arm 410. As described above, the second joint part 440 may receive torque due to the weight of the second arm and the weight of the source assembly 110. When the second arm 420 is fixed relative to the first arm 410, the magnetic brake 530 may provide a force that can offset the torque that the second joint part 440 receives due to the weight of the second arm and the weight of the source assembly 110. In addition, the magnetic brake 530 may be released based on a control signal of the controller 300, and the joint motor 540 may begin to rotate. More specifically, when the magnetic brake 530 is released, the torque that the magnetic brake 530 was offsetting may be controlled to be offset by the joint motor 540. The controller may use the torque sensor to measure the torque that the magnetic brake 530 was offsetting. The torque that the magnetic brake 530 was offsetting may be torque due to the weight of at least one of the second arm 420 and the source assembly 110. The controller may allow the joint motor 540 to generate torque simultaneously as the magnetic brake 530 is released, thereby allowing the second arm 420 to maintain a fixed state. At this time, the gas spring 441 may prevent sudden movement of the second arm 420. In addition, the gas spring 441 may provide a force (torque) that supports at least one of the source assembly 110 and the second arm 420 to prevent movement thereof in the direction of gravity. Therefore, at least one of the magnetic brake 530 and the joint motor 540 may offset the torque due to the weight of at least one of the source assembly 110 and the second arm 420 with little force. In this state, as the controller 300 changes the torque generated by the joint motor 540, the second arm 420 may move relative to the first arm 410.

The joint motor 540 may be a component that provides a driving force based on electrical energy. In the second joint part 440 on which the smart actuator 500 is mounted, the joint motor 540 may provide a driving force for the second arm 420 to rotate relative to the first arm 410.

The harmonic drive 550 may be a type of decelerator. The harmonic drive 550 may be a decelerator that uses the principle of epicyclic gearing using a bend of a rigid body. The harmonic drive 550 is favorably used in miniaturization of a mechanical device, such as a highly rigid mechanical device or a high-output mechanical device, because a basic deceleration ratio is high, and there is almost no backlash. The harmonic drive may rotate the driving shaft of the smart actuator 500 based on the driving force provided by the joint motor 540.

In addition, although not illustrated in FIG. 5, the smart actuator 500 may include a torque sensor. The torque sensor may be a component that measures torque applied from the outside to the driving shaft of the joint part 450. For example, the smart actuator 500 may be in a state in which it is not able to move due to the magnetic brake 530. At this time, the torque sensor may measure at least one of the torque applied to the joint part 450 by a user and the torque applied to the joint part 450 due to gravity. In addition, the smart actuator 500 may be in a state in which it is able to move due to the joint motor 540. At this time, the torque sensor may measure at least one of the torque applied to the joint part 450 due to gravity, the torque applied to the joint part 450 by the joint motor 540, and the torque applied to the joint part 450 due to an outer force.

As described above, the medical imaging device 100 may include the controller 300. The controller 300 may be a component for controlling the joint part 450 including at least one of the first joint part 430 and the second joint part 440.

The controller 300 may control the second joint part 440 to rotate the second arm 420 relative to the first arm 410 based on at least one of the torque applied to the joint part and a user input. A process of controlling the second joint part 440 based on the torque applied to the joint part will be described below.

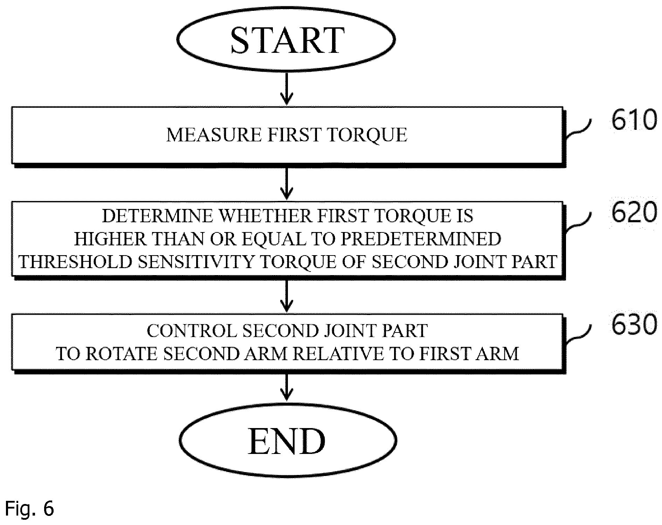

FIG. 6 is a flowchart for describing an operation of the medical imaging device according to one embodiment of the present disclosure.

Steps in FIG. 6 may be performed when the second arm 420 is stationary relative to the first arm 410. More specifically, the second arm 420 may be stationary relative to the first arm 410 due to the magnetic brake 530 included in the second joint part 440.

The controller 300 may perform a step of measuring a first torque applied to the second joint part 440 (610). More specifically, the second joint part 440 may include the smart actuator 500, and the controller 300 may perform the step of measuring the first torque (610) based on at least one of the torque sensor and the joint encoder 520 included in the smart actuator 500.

The first torque may be a force applied to the second joint part 440 by an outer force in a state in which the second arm 420 is stationary relative to the first arm 410. The second arm 420 being stationary relative to the first arm 410 may indicate a state in which the torque caused by the second arm 420 and the source assembly 110 is offset by at least one of the magnetic brake 530 and the gas spring 441. At this time, an additional outer force, such as a force exerted by a user, may be applied to the second arm 420, and the first torque may be generated at the second joint part 440.

The controller 300 may perform a step of determining whether the first torque is higher than or equal to a predetermined threshold sensitivity torque of the second joint part (620). The predetermined threshold sensitivity torque of the second joint part may be set by a user or may be automatically determined based on a predetermined algorithm. The threshold sensitivity torque of the second joint part may be related to a force necessary for a user to move the source arm 140. The threshold sensitivity torque of the second joint part may be changeable. The lower the threshold sensitivity torque of the second joint part, the smaller the initial force necessary for a user to move the second arm 420 relative to the first arm 410. In addition, the higher the threshold sensitivity torque of the second joint part, the greater the initial force necessary for a user to move the second arm 420 relative to the first arm 410.

One of a plurality of predetermined candidate threshold sensitivity torques may be selected as the threshold sensitivity torque of the second joint part. The plurality of candidate threshold sensitivity torques may include five different torques. For example, the plurality of candidate threshold sensitivity torques may correspond to one of very sensitive, sensitive, moderate, insensitive, and very insensitive. The size of the candidate threshold sensitivity torque may gradually increase from very sensitive to very insensitive. One of the plurality of predetermined candidate threshold sensitivity torques may be selected based on a user selection input. Although the force required to move the second arm 420 is smaller when the threshold sensitivity torque is lower, there may be a possibility that the second arm 420 moves incorrectly. Although the force required to move the second arm 420 is greater when the threshold sensitivity torque is higher, there may be no possibility of incorrect movement of the second arm 420.

According to various embodiments of the present disclosure, the medical imaging device 100 may select one of the candidate threshold sensitivity torques based on user identification information. More specifically, a user may register identification information in the medical imaging device 100. The medical imaging device 100 may allow only a user whose user identification information is registered in the medical imaging device 100 to use the medical imaging device 100. The medical imaging device 100 may store the threshold sensitivity torque of the second joint part that corresponds to identification information of each user. Accordingly, when a user inputs user identification information to the medical imaging device 100 to use the medical imaging device 100, the medical imaging device 100 may automatically select one of the plurality of candidate threshold sensitivity torques.

The medical imaging device 100 may perform the following steps to store the threshold sensitivity torque of the second joint part that corresponds to identification information of each user. The medical imaging device 100 may receive a threshold sensitivity torque together when receiving user identification information. In addition, the medical imaging device 100 may automatically determine a threshold sensitivity torque based on at least one of gender, age, and weight of the user. In addition, the medical imaging device 100 may output a message that instructs the user to comfortably apply a force to rotate the second arm 420 relative to the first arm 410 as a test. For example, the medical imaging device 100 may output a message that instructs to comfortably apply a force to "lift" the second arm 420. The user may apply a force to the second arm 420. The medical imaging device 100 may measure a torque applied to the second joint part 440 based on the force applied to the second arm 420 by the user. The torque applied to the second joint part 440 may be a net torque applied to the second joint part 440. However, the present disclosure is not limited thereto, and the torque applied to the second joint part 440 may be a torque applied to the second joint part 440 by the user.

The medical imaging device 100 may select the candidate threshold sensitivity torque that is closest to the measured torque as the threshold sensitivity torque of the second joint part. Alternatively, the medical imaging device 100 may select the candidate threshold sensitivity torque that is higher than the measured torque and closest to the measured torque as the threshold sensitivity torque of the second joint part. In addition, the medical imaging device 100 may select the candidate threshold sensitivity torque that is lower than the measured torque and closest to the measured torque as the threshold sensitivity torque of the second joint part. In addition, the medical imaging device 100 may determine the measured torque as the threshold sensitivity torque of the second joint part.

Although only the threshold sensitivity torque of the second joint part has been described above, a threshold sensitivity torque may also be set for the first joint part 430. Since the same description may apply to the threshold sensitivity torque of the first joint part, repeated description will be omitted.

When the first torque is higher than or equal to the threshold sensitivity torque of the second joint part 440, the controller 300 may perform a step of controlling the second joint part to rotate the second arm relative to the first arm 410 (630). A direction of rotation of the second arm 420 relative to the first arm 410 may be the same as a direction of the force applied to the second arm 420 by the user. For example, if the user has applied an upward force to the second arm 420 in FIG. 4, the second arm 420 may rotate counterclockwise. In addition, if the user has applied a downward force to the second arm 420, the second arm 420 may rotate clockwise.

When the user has applied a force to the second arm 420, torque may be applied not only to the second joint part 440 but also to the first joint part 430. The controller 300 may control only one of the first joint part 430 and the second joint part 440 to move. However, the present disclosure is not limited thereto, and the controller 300 may control the first joint part 430 and the second joint part 440 to move simultaneously. The controller 300 may determine a mode as one of a mode in which only one of the first joint part 430 and the second joint part 440 moves and a mode in which both the first joint part 430 and the second joint part 440 move, based on a user input.

In the case of the mode in which only one of the first joint part 430 and the second joint part 440 moves, the controller 300 may move only the first joint part 430 or move only the second joint part 440 based on whether the user has applied a force to the second arm 420 or applied a force to the first arm 410. For example, when the user has applied a force to the second arm 420, both the first joint part 430 and the second joint part 440 of the medical imaging device 100 may receive torque. Therefore, when a second joint part torque measured at the second joint part 440 is higher than or equal to a predetermined threshold start torque, the medical imaging device 100 may determine to rotate the second joint part 440 regardless of a first joint part torque measured at the first joint part 430. That is, the first arm 410 may be fixed relative to the main body 130, and the second arm 420 may move relative to the first arm 410. When it is determined to move the second arm 420, the process of FIG. 6 may be performed.

In addition, when the second joint part torque measured at the second joint part 440 is lower than or equal to the predetermined threshold start torque, and the first joint part torque measured at the first joint part 430 is higher than or equal to the threshold start torque, the medical imaging device 100 may determine to rotate the first joint part 430. That is, the first arm 410 may move relative to the main body 130, and the second arm 420 may be fixed relative to the first arm 410. In this way, since a user can move the first arm 410 or the second arm 420 as intended by moving one of the first joint part 430 and the second joint part 440, and this is intuitive, it may be convenient for the user. However, the present disclosure is not limited thereto.

Step (630) may include the following steps. In order to prevent the second arm 420 from accelerating extremely fast, the controller 300 may control the second arm 420 to accelerate at a predetermined maximum angular acceleration or lower in a state in which the second arm 420 is standing still. In addition, in order to prevent the second arm 420 from moving extremely fast, the controller 300 may control the second arm 420 to move at a predetermined maximum constant angular velocity or lower after causing the second arm 420 to accelerate from the standstill state. In addition, the controller 300 may allow the second arm 420 to move when the user is applying a force to the second arm 420 and may prevent the second arm 420 from moving again when the user is not applying a force to the second arm 420. When the second arm 420 is moving, the torque that the user has to apply to the second arm 420 may be lower than or equal to the threshold sensitivity torque. In order to prevent the second arm 420 from decelerating extremely fast, the controller 300 may control the second arm 420 to decelerate at a predetermined maximum angular acceleration or lower when the second arm 420, which was moving, stops. Therefore, it may be possible to prevent the user from getting startled at sudden movement or uncontrollable angular velocity of the second arm 420.

Since the second arm 420 and the source assembly 110 are coupled to the second joint part 440, the torque caused by the weights of the second arm 420 and the source assembly 110 may be applied to the second joint part 440. The torque caused by the weights of the second arm 420 and the source assembly 110 may be applied to the second joint part 440 even while the second arm 420 is moving. The torque applied to the second joint part 440 due to the second arm 420 and the source assembly 110 may be may be determined by a predetermined function. The predetermined function may output an output torque applied to the second joint part 440 with at least one of the weight of the second arm 420, the weight of the source assembly 110, the angle of the first arm 410 relative to the ground, the angle of the second arm 420 relative to the first arm 410, the length of the second arm 420, the length of the gas spring, and the force that the gas spring provides as a variable. The output torque may be the torque caused by the weights of the second arm 420 and the source assembly 110. The controller 300 may allow the smart actuator included in the second joint part 440 to generate torque in the opposite direction of the output torque determined by the predetermined function and may control the second arm 420 to move at a constant angular velocity relative to the first arm 410. For example, when the second arm moves at a constant angular velocity, the torque generated by the smart actuator may be as follows.
1) TOUT = -T1 - T2 (when the torque applied to the second arm by the user is in the same direction as the torque caused by the weights of the second arm 420 and the source assembly 110)
2) TOUT = -T1 + T2 (when the torque applied to the second arm by the user is in a different direction from the torque caused by the weights of the second arm 420 and the source assembly 110)

Here, TOUT may be the size of the torque generated by the smart actuator. T1 may be the torque caused by the weights of the second arm 420 and the source assembly 110. T2 may be the torque applied to the second arm (or the second joint part 440) by the user.

Using the threshold sensitivity torque of the second joint part that may be changed in this way, the user can comfortably move the second arm 420 relative to the first arm 410 regardless of the muscular strength of the user. That is, since the threshold sensitivity torque is determined according to the user, the user can freely control the arms of the medical imaging device 100 regardless of the muscular strength of the user.

A process of controlling the second joint part 440 to rotate the second arm 420 relative to the first arm 410 based on a user input will be described below. Here, the user input may indicate an input of a user's intention on the input part 350 such as a button or a touchscreen, instead of a force directly applied to the second arm 420 by the user.

The controller 300 may perform a step of controlling the second joint part so that a predetermined angle is formed between the first arm 410 and the second arm 420 based on a user input on a button related to joint movement. For example, the predetermined angle may be 90° or more and 180° or less. In addition, the predetermined angle may indicate an angle between the first arm 410 and the second arm 420 when the second arm 420 is almost parallel to the ground.

Referring to FIG. 4, the button related to joint movement may be a physical button. The button related to joint movement may be included in at least two of the second joint part 440, the second arm 420, and the source assembly 110. For example, the button related to joint movement may be located on a left side or a right side of the second joint part 440. In addition, the button related to joint movement may be located on at least one of an upper side, a left side, a right side, and a lower side of the second arm 420. In addition, the button related to joint movement may be located on at least one of a front side, an upper side, a left side, a right side, and a lower side of the source assembly 110.

In addition, the button related to joint movement may be a button displayed on a graphical user interface (GUI) such as a touchscreen. The button related to joint movement may be located on at least one of a sub display located on the source assembly 110 and the main display 150 located on the main body 130. The sub display located on the source assembly 110 may be located on a front surface of the source assembly 110. However, the present disclosure is not limited thereto.

The first arm 410 and the second arm 420 may each be in a folded state to prevent the first arm 410 and the second arm 420 from colliding with a nearby object during movement of the medical imaging device 100. For example, the medical imaging device 100 may move in a posture shown in FIG. 1. The second arm 420 may be in a state in which it is almost perpendicular to the ground. Therefore, by minimizing the torque applied to the second joint part 440 due to the source assembly 110 and the second arm 420, damage to the second joint part 440 due to impact during movement can be prevented. In addition, collision of the source assembly 110 with a nearby object can be minimized.

After the medical imaging device 100 is placed near a patient, when an input on a button is received from a user, the medical imaging device 100 may allow the second arm 420 to have a predetermined angle relative to the first arm 410. That is, from the posture of FIG. 1, the second arm 420 may rotate counterclockwise based on the second joint part 440, and the predetermined angle may be formed between the first arm 410 and the second arm 420. With such a process, the second arm 420 may have an unfolded posture relative to the first arm 410. For example, the unfolded posture of the second arm 420 may be that shown in FIG. 4. The unfolded posture of the second arm 420 may indicate a state in which the second arm 420 is parallel to the ground. However, the present disclosure is not limited thereto. The angular velocity of movement of the second arm 420 may be predetermined. In addition, the angular velocity of movement of the second arm 420 may be changed by user setting. Since there is no need for the user to lift the second arm 420, user convenience can be enhanced.

In addition, when an input on the button is received from the user after imaging ends, the medical imaging device 100 may rotate the second arm clockwise so that the second arm 420 has the posture shown in FIG. 1 again. That is, the second arm 420 may return to a posture in which it is movable. The movable posture may be a posture in which the second arm 420 is folded relative to the first arm 410. Since there is no need for the user to move the second arm to the movable posture again, user convenience can be enhanced.

The medical imaging device 100 may have a plurality of buttons related to joint movement, and the user may allow the second arm 420 to move relative to the first arm 410 with minimum movement of the user.

The medical imaging device 100 may include the source assembly 110. One end of the second arm 420 may be coupled to the second joint part 440. In addition, the other end of the second arm 420 may be coupled to the source assembly 110. The source assembly 110 may include a second transceiver. The second transceiver may include a second transmitter and a second receiver. The second transceiver may be located on a surface of the source assembly 110 that is located in a direction in which radiation exits. The second transceiver may be located on a surface of the source assembly 110 that is perpendicular to the direction in which radiation exits. The second transceiver may communicate with a first transceiver. The first transceiver and the second transceiver may communicate with each other using UWB.

The medical imaging device 100 may include the detector 120. The detector 120 may receive radiation radiated from the source assembly 110 and may generate a medical image. The detector 120 may include the first transceiver transmitting and receiving a signal to and from the second transceiver. The first transceiver may be a component for wirelessly communicating with the second transceiver included in the source assembly 110. The first transceiver may include a first transmitter and a first receiver. The detector 120 may include a plurality of first transceivers. The first transceivers may be located on a surface of the detector 120 that receives radiation. The first transceivers may be arranged at a corner of the detector 120. The first transceivers may be located on a left side surface and a right side surface of the detector 120. For example, two first transceivers may be disposed on each of the left side surface and the right side surface of the detector 120. When the plurality of first transceivers are arranged on the detector 120 in this way, the medical imaging device 100 may precisely align the detector 120 and the source assembly 110. However, the present disclosure is not limited thereto, and the first transceivers may be located in the vicinity of vertices of the detector 120 having a quadrangular shape.

Based on the first transceiver and the second transceiver, the controller 300 may perform a step of controlling the joint part so that a radiation irradiation direction of the source assembly 110 becomes perpendicular to a radiation reception surface of the detector 120.

More specifically, the main controller 300 may perform a step of outputting a message with guidance on adjusting a radiation irradiation region of the source assembly 110 and a detector region based on alignment information. Based on at least one of a 3D camera, the first transceiver, and the second transceiver, the main controller 300 may determine alignment information related to at least one of the direction, distance, and angle of movement of the source assembly 110 that is necessary to align the detector 120 and the source assembly 110. The main controller 300 may obtain alignment information to perform the step of outputting the message. The alignment information may be information for aligning the source assembly 110 and the detector 120. Here, aligning the detector 120 and the source assembly 110 may mean letting the irradiation region and the detector region coincide or letting a line connecting the center of the detector and the center of the source assembly 110 be parallel to the radiation irradiation direction.

The main controller 300 may obtain at least one of posture information of the detector 120 and posture information of the source assembly 110 based on the first transceiver and the second transceiver. The main controller 300 may further use the 3D camera to obtain at least one of the posture information of the detector 120 and the posture information of the source assembly 110. Alternatively, the main controller 300 may further use the 3D camera to revise at least one of the posture information of the detector 120 and the posture information of the source assembly 110.

The main controller 300 may perform a step of obtaining alignment information for adjusting an angle of the source assembly 110 so that the radiation irradiation direction of the source assembly 110 becomes perpendicular to the radiation reception surface of the detector 120 based on at least one of the posture information of the detector 120 and the posture information of the source assembly 110. The posture information of the detector 120 may be obtained based on at least one of the gyro sensor and the first transceiver included in the detector 120. The detector 120 may indirectly measure the posture information by triangulation using the first transceiver and the second transceiver. The posture information of the detector 120 may include at least one of a degree of inclination (slope) of the detector 120 relative to the source assembly 110 or the ground and a distance from the detector 120 to the source assembly 110 or the ground. The posture information of the detector 120 may include a degree of rotation of the detector 120 based on at least one of a first axis parallel to the ground, a second axis parallel to the ground and perpendicular to the first axis, and a third axis perpendicular to the ground. The posture information of the detector 120 may include a distance from one point of the source assembly 110 to one point of the detector 120. The posture information of the detector 120 may include coordinates from one of one point of the source assembly 110 and one point of the main body 130 to one point of the detector 120. The one point of the source assembly 110 may be one of the center of the source assembly 110 and the center of a front surface of the source assembly 110. In addition, the one point of the detector 120 may be the center of the radiation reception surface of the detector 120. However, the one point of the main body 130, the one point of the source assembly 110, and the one point of the detector 120 are not limited to the above description as long as they are points included in the main body 130, the source assembly 110, and the detector 120, respectively. The posture information of the detector 120 may include a slope of the radiation reception surface of the detector 120 relative to the front surface of the source assembly 110.

The main controller 300 included in the medical imaging device 100 may obtain the posture information of the source assembly 110 based on at least one of the gyro sensor and the second transceiver. The posture information of the source assembly 110 may include at least one of a degree of inclination (slope) of the source assembly 110 relative to the detector 120 or the ground and a distance from the source assembly 110 to the detector 120 or the ground. The main controller 300 may directly measure the posture information of the source assembly 110 based on the gyro sensor or may indirectly measure the posture information of the source assembly 110 by triangulation using the first transceiver and the second transceiver. The posture information of the source assembly 110 may include a degree of rotation of the source assembly 110 based on at least one of the first axis, the second axis, and the third axis. The first axis, the second axis, and the third axis may be axes perpendicular to one another. The posture information of the source assembly 110 may include a distance from one of one point of the detector 120 and one point of the main body 130 to one point of the source assembly 110. The posture information of the source assembly 110 may include coordinates from one point of the source assembly 110 to one point of the detector 120. The posture information of the source assembly 110 may include a slope of the front surface of the source assembly 110 relative to the radiation reception surface of the detector 120.

The medical imaging device 100 may determine alignment information based on at least one of the posture information of the detector 120 and the posture information of the source assembly 110. In addition, the medical imaging device 100 may perform a step of controlling the joint part so that at least one of the first arm 410 and the second arm 420 is moved based on the alignment information, and the radiation irradiation direction of the source assembly 110 becomes perpendicular to the radiation reception surface of the detector 120. Since the medical imaging device 100 automatically moves at least one of the first arm 410 and the second arm 420 so that the radiation irradiation direction of the source assembly 110 becomes perpendicular to the radiation reception surface of the detector 120 in this way, there is almost no need for the user to manipulate the source assembly 110. In addition, there is also no need to adjust the position of the source assembly 110 while looking at a sensor value displayed on the display to place the source assembly 110 at an accurate position. Therefore, the medical imaging device 100 can maximize user convenience.

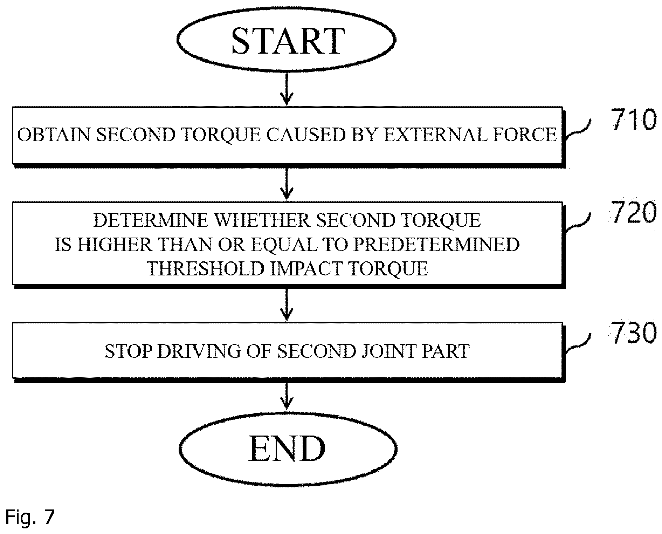

FIG. 7 is a flowchart showing an operation of the medical imaging device according to one embodiment of the present disclosure.

The controller 300 may perform a step of obtaining a second torque caused by an external force while the second arm 420 moves relative to the first arm 410 due to driving of the second joint part 440 (710). Due to the control of the controller 300, the second joint part 440 may allow the second arm 420 to move at a constant angular velocity relative to the first arm 410. Although torque may act on the second joint part 440 due to the weight of the second arm 420 and the weight of the source assembly 110, the smart actuator included in the second joint part 440 may offset the torque caused by the weight of the second arm 420 and the weight of the source assembly 110 and may allow the second arm 420 to move at a constant angular velocity.

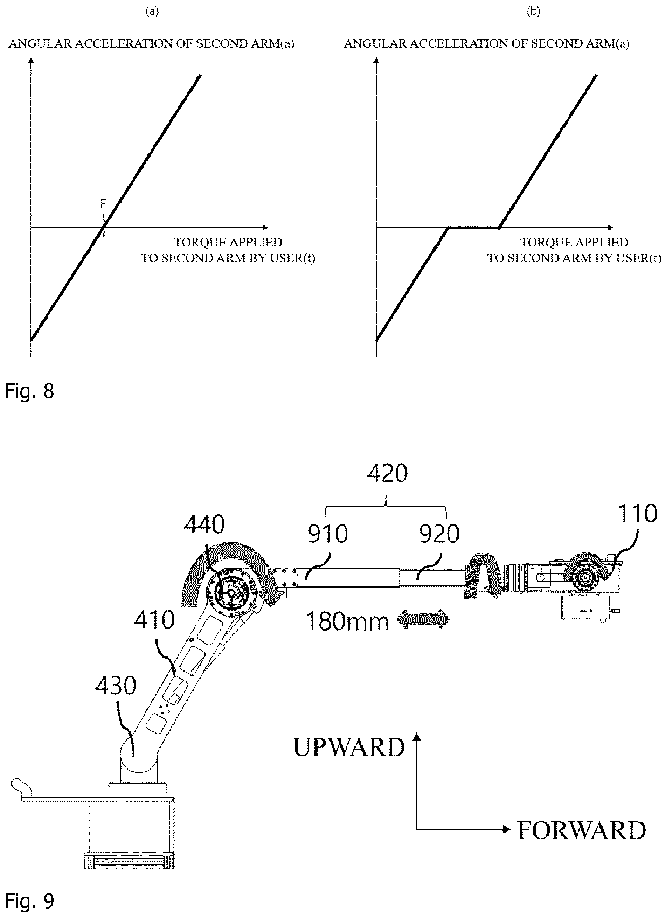

Alternatively, due to the control of the controller 300, the second joint part 440 may allow the second arm 420 to move relative to the first arm 410 at an angular acceleration proportional to the torque that the user applies to the second arm 420 (or the second joint part 440). Movement of the second arm 420 will be described with reference to FIG. 8.

FIG. 8 is a view for describing angular acceleration of the second arm of the present disclosure.

The x-axis of FIG. 8 may indicate the torque that the user applies to the second arm, and the y-axis may indicate the angular acceleration of the second arm. FIG. 8 shows graphs when the angular acceleration of the second arm is almost 0.

Referring to FIG. 8A, a torque t that the user applies to the second arm 420 may be proportional to an angular acceleration a of movement of the second arm 420. In a state in which the second arm 420 is standing still, the second arm 420 may start to move relative to the first arm 410 due to the process shown in FIG. 6. Then, the user has to continuously exert force on the second arm 420 for the controller 300 to maintain the movement of the second arm 420. For example, the relational expression between the torque t that the user applies to the second arm 420 and the angular acceleration a may be as follows. $a = K * \left(t-F\right)$

Here, t may be the torque t that the user applies to the second arm 420, and a may be the angular acceleration of movement of the second arm 420. K may be a predetermined proportional constant. In addition, F may be a predetermined constant. F may be a threshold movement torque that the user has to apply to the second arm 420 to move the second arm 420 at a constant angular velocity or a positive angular acceleration. The threshold movement torque F may be equal to the threshold sensitivity torque or may be lower than the threshold sensitivity torque. F may serve as a type of virtual frictional force. Generally, since the user intuitively knows that there is a frictional force when moving an object, the medical imaging device 100 of the present disclosure may use virtual F as a control variable for the user to intuitively move the second arm 420.

Referring to FIG. 8B, the relational expression between the torque t that the user applies to the second arm 420 and the angular acceleration a may be as follows. $a = K * \left(t-F\right) ,$ when t is smaller than F.
a = 0, when t is greater than or equal to F and smaller than or equal to MF. $a = K * \left(t-MF\right) ,$ when t is greater than MF.

Unlike in FIG. 8A, in FIG. 8B, when the torque that the user applies to the second arm 420 is greater than or equal to F and smaller than or equal to MF, the second arm 420 may move at a constant angular velocity. Here, F and MF may be predetermined constants. When the angular acceleration of the second arm 420 changes continuously, the user may have difficulty in controlling the movement of the second arm 420. Therefore, the medical imaging device 100 of the present disclosure may control the movement of the second arm 420 as in FIG. 8B.

However, the movement of the second arm 420 is not limited to that shown in FIG. 8. The second arm 420 may always move at a constant angular velocity. In addition, the angular acceleration of the second arm 420 may be limited to a predetermined maximum angular acceleration or lower. In addition, the angular acceleration of the second arm 420 may be limited to a predetermined minimum angular acceleration or higher.

Referring back to FIG. 7, in step (710), the second torque may indicate a torque that is different from the torque that the user applies to the second arm 420 to move the second arm 420. The second torque may be torque generated when contact unintended by the user occurs between an external object and the second arm 420 or the first arm 410. That is, the second torque may be torque generated when the second arm 420 or the first arm 410 collides with an external object.

The controller 300 may measure an absolute value of a change amount per unit time of the torque applied to the second arm. When the absolute value of the change amount per unit time is higher than or equal to a predetermined impact detection time determination threshold change torque, the controller 300 may determine that impact has occurred on the second arm 420. The controller 300 may determine the second torque by subtracting the torque t that the user applies to the second arm right before the time at which the impact occurs from a torque at applied to the second arm 420 by an external object and the user right after the time at which the impact occurs. That is, the second torque may indicate the torque applied to the second arm by the external object. The controller 300 may measure the second torque using a sensor.

The controller 300 may perform a step of determining whether the second torque is higher than or equal to a predetermined threshold impact torque (720). The threshold impact torque is a predetermined value and may be a value for determining whether the impact has actually occurred. The threshold impact torque may be changeable. For example, the threshold impact torque may be directly proportional to the threshold sensitivity torque. Therefore, since the threshold impact torque and the threshold sensitivity torque have a direct proportional relationship, the possibility of the controller 300 misjudging that impact has occurred on the second arm 420 may become very low.

When the second torque is higher than or equal to the predetermined threshold impact torque, the controller 300 may perform a step of stopping driving of the second joint part 440 (730). Therefore, when the second arm 420 is in contact with an external object, the medical imaging device 100 of the present disclosure may immediately stop to prevent additional damage to the external object. In addition, in this way, damage to the components of the medical imaging device 100 such as the second arm 420 and the source assembly 110 can also be prevented.

According to various embodiments of the present disclosure, the medical imaging device 100 may include a distance sensor. The distance sensor may be a component for determining whether an external object is approaching. The distance sensor may be located on at least one of an upper side and a lower side of the source assembly 110. In addition, the distance sensor may be included in at least one of the first arm 410 and the second arm 420. For example, the distance sensor may be located on at least one of an upper side and a lower side of at least one of the first arm 410 and the second arm 420. The controller 300 may use the distance sensor to measure a distance between an external object and the medical imaging device 100. When the measured distance is smaller than or equal to a threshold distance, the controller 300 may stop driving of the second joint part 440. Accordingly, a situation in which the medical imaging device 100 comes into contact with an external object can be prevented.

The medical imaging device 100 may stop at least one of the first arm 410 and the second arm 420 due to impact from an external object and then may continue an operation that was being performed manually or automatically. For example, when impact is detected during an operation of unfolding or folding the second arm 420 relative to the first arm 410, the medical imaging device 100 may stop the operation of moving the second arm 420. Then, when the user presses a collision release button, the medical imaging device 100 may continue the operation of unfolding or folding the second arm 420 relative to the first arm 410. In addition, based on the distance sensor and the torque sensor, the medical imaging device 100 may obtain a signal indicating that there is no external object. The medical imaging device 100 may continue the unfolding or folding operation based on the signal indicating that there is no external object.

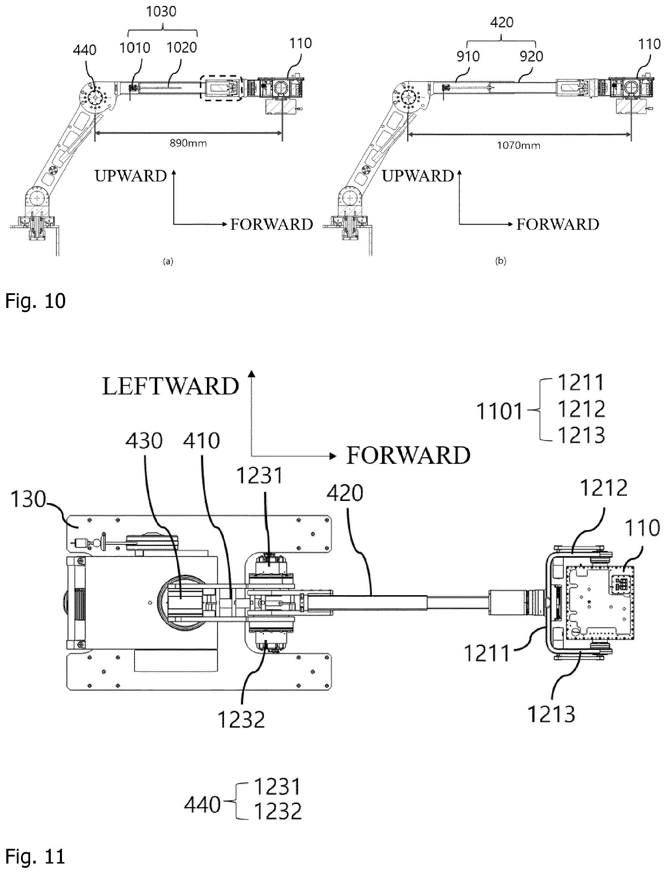

FIG. 9 is a view for describing a degree of freedom of an arm of the medical imaging device according to one embodiment of the present disclosure. FIG. 10 is a view for describing a component for moving the arm of the medical imaging device according to one embodiment of the present disclosure.

FIGS. 9 and 10 show side views of the medical imaging device 100. Description of parts shown in FIGS. 9 and 10 that have already been described above will be omitted.

The second arm 420 may include a second-first arm 910 and a second-second arm 920. The second-first arm 910 may have one end coupled to the second joint part 440. The second-first arm 910 may have a tubular shape. A cross-section of the second-first arm 910 along a surface perpendicular to the longitudinal direction of the second-first arm 910 may be one of circular, quadrangular, hexagonal, and octagonal. A space may be formed inside the second-first arm 910.

The second-second arm 920 may have at least one portion inserted into the space formed inside the second-first arm 910. In addition, the second-second arm 920 may move along the second-first arm 910. The second arm 420 may be able to extend or contract due to the second-first arm 910 and the second-second arm 920. For example, when the second-second arm 920 is maximally inserted into the second-first arm 910, the second arm 420 may have a minimum length. The minimum length may be 890 mm, for example. In addition, when the second-second arm 920 is minimally inserted into the second-first arm 910, the second arm 420 may have a maximum length. The maximum length may be 1070 mm, for example. The second-second arm 920 may move 180 mm relative to the second-first arm 910. Since the second arm 420 extends or contracts in this way, even when space around a patient is not sufficient, the second arm 420 may extend, and the source assembly 110 may be placed near the patient. In particular, in a typical hospital room, space to the left and right of a patient table is very narrow, and the medical imaging device 100 is not able to enter. Therefore, a medical image should be captured by placing the medical imaging device 100 in front of or behind the patient table. It has been difficult to place the source assembly 110 close to the patient in some cases because the front-rear length of the patient table is longer than the left-right width thereof, but in the medical imaging device 100 of the present disclosure, since the second arm 420 extends, there is an advantage that the source assembly 110 can be placed near a patient.

The second arm 420 may include the telescopic arm driving part 1030. The telescopic arm driving part 1030 may be coupled to an inner portion of the second-first arm 910. The telescopic arm driving part 1030 may be a component that provides a driving force for the second-second arm 920 to move relative to the second-first arm 910.

The telescopic arm driving part 1030 may include a telescopic arm motor 1010 and a telescopic arm shaft 1020. The telescopic arm motor 1010 may rotate the telescopic arm shaft 1020 based on a signal of the controller 300. Referring to FIG. 10, the telescopic arm shaft 1020 may rotate about an axis parallel to the front-rear direction. The telescopic arm shaft 1020 may rotate about an axis parallel to a direction in which the second arm 420 extends. A screw thread may be formed on an outer circumferential surface of the telescopic arm shaft 1020. The screw thread formed on the outer circumferential surface of the telescopic arm shaft 1020 may be coupled to a screw hole formed at one end of the second-second arm 920. Therefore, due to rotation of the telescopic arm shaft 1020, the second-second arm 920 may move forward or upward relative to the second-first arm 910.

The controller may control the second-second arm to move relative to the second-first arm based on one of a user input on a button related to a telescopic function and a force that the user applies to the second-second arm.

More specifically, the user may extend the second arm 420 or contract the second arm 420 using the button related to the telescopic function. For example, the user may press an extension button to extend the second arm 420 and may press a contraction button to contract the second arm 420. However, the present disclosure is not limited thereto, and the extension button and the contraction button may be configured as one button. For example, the second arm 420 may extend when a button is pressed one time, and the second arm 420 may contract when the same button is pressed one more time.

Since the second-second arm 920 automatically moves in this way, there may be no need for the user to extend or contract the second arm 420 using muscular strength. In addition, the medical imaging device 100 may control the joint part so that the radiation irradiation direction of the source assembly 110 automatically becomes perpendicular to the radiation reception surface of the detector 120. Therefore, user convenience can be enhanced.

As described above, in the medical imaging device 100 of the present disclosure, at least one of the threshold sensitivity torque, the threshold start torque, the threshold movement torque F, and the threshold impact torque may be determined based on the length of the second arm 420.

For example, at least one of the threshold sensitivity torque, the threshold start torque, the threshold movement torque F, and the threshold impact torque may increase with an increase in the length of the second arm 420. The torque that the weight of the source assembly 110 applies to the second joint part 440 may increase with an increase in the length of the second arm 420. In addition, the user may move the second arm 420 while holding the vicinity of the source assembly 110 to make the radiation irradiation direction of the source assembly 110 face a patient. At this time, when the second arm 420 is long, the torque that the user applies to the second joint part 440 may increase. Therefore, the controller 300 may set at least one of the threshold sensitivity torque, the threshold start torque, the threshold movement torque F, and the threshold impact torque to be higher with an increase in the length of the second arm 420. Therefore, the same user experience can always be maintained regardless of the length of the second arm 420.

However, the present disclosure is not limited thereto, and at least one of the threshold sensitivity torque, the threshold start torque, the threshold movement torque F, and the threshold impact torque may be irrelevant to the length of the second arm 420.

The telescopic arm driving part 1030 may also serve as a brake. That is, due to the telescopic arm driving part 1030, there may be no movement of the second-second arm 920 relative to the second-first arm 910 caused by an external object. This is because, due to the telescopic arm driving part 1030, a very large external force is required for movement of the second-second arm 920 relative to the second-first arm 910. That is, in most cases, movement of the second-second arm 920 relative to the second-first arm 910 may be possible due to the telescopic arm driving part 1030.

Since the telescopic arm driving part 1030 serves as a brake in this way, a case in which the second-second arm 920 moves relative to the second-first arm 910 unexpectedly can be prevented. Therefore, safety of the medical imaging device 100 may increase.

The controller 300 may control the second-second arm to move relative to the second-first arm based on the force that the user applies to the second-second arm. More specifically, the second arm 420 may contract or extend based on a measured force of the user. For example, the controller 300 may perform a step of measuring a linear force applied to the second arm 420. The telescopic arm driving part 1030 may include a force sensor. The telescopic force sensor may measure a linear force that the user applies to the second-second arm 920 relative to the second-first arm 910. More specifically, when the user applies a forward or rearward force while holding the second-second arm 920, the telescopic force sensor may detect the linear force applied by the user. The direction of the linear force may be one of forward and rearward. The controller 300 may receive the force measured by the telescopic force sensor.

The controller 300 may perform a step of determining whether the linear force is greater than or equal to a predetermined threshold sensitivity force. The predetermined threshold sensitivity force may be set by the user or may be automatically determined based on a predetermined algorithm. The threshold sensitivity force may be relevant to a force necessary for the user to move the second-second arm 920 relative to the second-first arm 910. The threshold sensitivity force may be changeable. The smaller the threshold sensitivity force, the smaller the initial force necessary for a user to move the second-second arm 920 relative to the second-first arm 910. In addition, the greater the threshold sensitivity force, the greater the initial force necessary for a user to move the second-second arm 920 relative to the second-first arm 910.

One of a plurality of predetermined candidate threshold sensitivity forces may be selected as the threshold sensitivity force. The plurality of candidate threshold sensitivity forces may include five different forces. For example, the plurality of candidate threshold sensitivity forces may correspond to one of very sensitive, sensitive, moderate, insensitive, and very insensitive. The size of the candidate threshold sensitivity force may gradually increase from very sensitive to very insensitive. One of the plurality of predetermined candidate threshold sensitivity forces may be selected based on a user selection input. Although the force required to move the second-second arm 920 is smaller when the threshold sensitivity force is smaller, there may be a possibility that the second-second arm 920 moves incorrectly. Although the force required to move the second-second arm 920 is greater when the threshold sensitivity force is greater, there may be no possibility of incorrect movement of the second-second arm 920.

According to various embodiments of the present disclosure, the medical imaging device 100 may select one of the candidate threshold sensitivity forces based on user identification information. More specifically, a user may register identification information in the medical imaging device 100. The medical imaging device 100 may allow only a user whose user identification information is registered in the medical imaging device 100 to use the medical imaging device 100. The medical imaging device 100 may store the threshold sensitivity force that corresponds to identification information of each user. Accordingly, when a user inputs user identification information to the medical imaging device 100 to use the medical imaging device 100, the medical imaging device 100 may automatically select one of the plurality of candidate threshold sensitivity forces.

The medical imaging device 100 may perform the following steps to store the threshold sensitivity force that corresponds to identification information of each user. The medical imaging device 100 may receive a threshold sensitivity force together when receiving user identification information. In addition, the medical imaging device 100 may automatically determine a threshold sensitivity force based on at least one of gender, age, and weight of the user. In addition, the medical imaging device 100 may output a message that instructs the user to apply a force to the second-second arm 920 relative to the second-first arm 910 as a test. For example, the medical imaging device 100 may output a message that instructs to comfortably "pull" or "press" the second-second arm 920 relative to the second-first arm 910. The user may apply a force to the second-second arm 920. The medical imaging device 100 may measure a force applied to the second-second arm 920 by the user.

The medical imaging device 100 may select the candidate threshold sensitivity force that is closest to the measured force as the threshold sensitivity force. Alternatively, the medical imaging device 100 may select the candidate threshold sensitivity force that is greater than the measured force and closest to the measured force as the threshold sensitivity force. In addition, the medical imaging device 100 may select the candidate threshold sensitivity force that is smaller than the measured force and closest to the measured force as the threshold sensitivity force. In addition, the medical imaging device 100 may determine the measured force as the threshold sensitivity force.

When the linear force is greater than or equal to the threshold sensitivity force, the controller 300 may perform a step of controlling the telescopic arm driving part 1030 to move the second-second arm 920 relative to the second-first arm 910. A direction of movement of the second-second arm 920 may be the same as a direction of movement of the linear force applied by the user. For example, if the user has applied a forward force to the second-second arm 920 in FIG. 9, the second-second arm 920 may move forward. In addition, if the user has applied a rearward force to the second-second arm 920, the second-second arm 920 may move rearward.

When the user has applied a force to the second-second arm 920, a force may be applied not only to the second-second arm 920 but also to the first joint part 430 and the second joint part 440. The controller 300 may control only one of the second-second arm 920, the first joint part 430, and the second joint part 440 to move. However, the present disclosure is not limited thereto, and the controller 300 may control the second-second arm 920, the first joint part 430, and the second joint part 440 to move simultaneously. The controller 300 may determine a mode as one of a mode in which only one of the second-second arm 920, the first joint part 430, and the second joint part 440 moves and a mode in which all of the second-second arm 920, the first joint part 430, and the second joint part 440 move, based on a user input.

In the case of the mode in which only one of the second-second arm 920, the first joint part 430, and the second joint part 440 moves, the controller 300 may move only the first joint part 430 or move only the second joint part 440 based on whether the user has applied a force to the second-second arm 920, applied a force to the second arm 420, or applied a force to the first arm 410. For example, when the user has applied a force to the second-second arm 920, not only the second-second arm 920, but also both the first joint part 430 and the second joint part 440 may receive force. Therefore, when the linear force applied to the second-second arm 920 is greater than or equal to a predetermined threshold start force, the medical imaging device 100 may determine to move the second-second arm 920 regardless of the torque measured at the first joint part 430 and the second joint part 440. That is, the first arm 410 may be fixed relative to the main body 130, the second arm 420 may be fixed relative to the first arm 410, and the second-second arm 920 may move relative to the second-first arm 910.

In addition, when the linear force measured at the second-second arm 920 is smaller than or equal to the predetermined threshold start force, and the torque measured at the second joint part 440 is higher than or equal to the threshold start torque, the medical imaging device 100 may determine to rotate the second joint part 440 regardless of the torque measured at the first joint part 430. That is, the first arm 410 may be fixed relative to the main body 130, and the second arm 420 may rotate relative to the first arm 410. In this way, since a user can move the second-second arm 920, the first arm 410, or the second arm 420 as intended by moving one of the second-second arm 920, the first joint part 430, and the second joint part 440, and this is intuitive, it may be convenient for the user. However, the present disclosure is not limited thereto.

The controller 300 may perform a step of obtaining an outer force caused by an external force while the second-second arm 920 moves relative to the second-first arm 910 due to the telescopic arm driving part 1030. The outer force may be obtained by the force sensor included in the telescopic arm driving part 1030. Due to control of the controller 300, the second-second arm 920 may move at a constant angular velocity relative to the second-first arm 910. The outer force may indicate a force different from the force that the user applies to the second-second arm 920 to move the second-second arm 920 or the force that the telescopic arm driving part 1030 applies to the second-second arm 920. The outer force may be a force generated when contact unintended by the user occurs between an external object and the second arm 420. That is, the outer force may be a force generated when the second arm 420 collides with an external object.

The controller 300 may measure an absolute value of a change amount per unit time of the outer force applied to the second-second arm 920. When the absolute value of the change amount per unit time is greater than or equal to a predetermined impact detection time determination threshold change force, the controller 300 may determine that impact has occurred on the second-second arm 920. The controller 300 may determine the outer force by subtracting the force that the user applies to the second-second arm 920 right before the time at which the impact occurs from the force applied to the second-second arm 920 by an external object and the user right after the time at which the impact occurs. The outer force may indicate the force applied by the external object. The controller 300 may measure the outer force using a sensor.

The controller 300 may perform a step of determining whether the outer force is greater than or equal to a predetermined threshold impact force. The threshold impact force is a predetermined value and may be a value for determining whether the impact has actually occurred. The threshold impact force may be changeable. For example, the threshold impact force may be directly proportional to the threshold sensitivity force. Therefore, since the threshold impact force and the threshold sensitivity force have a direct proportional relationship, the possibility of the controller 300 misjudging that impact has occurred on the second-second arm 920 may become very low.

When the outer force is greater than or equal to the predetermined threshold impact force, the controller 300 may perform a step of stopping driving of the telescopic arm driving part 1030. Therefore, when the second-second arm 920 is in contact with an external object, the medical imaging device 100 of the present disclosure may immediately stop to prevent additional damage to the external object. In addition, in this way, damage to the components of the medical imaging device 100 such as the second-second arm 920 and the source assembly 110 can also be prevented.

Referring to FIG. 9, the other end of the second arm 420 may be coupled to the source assembly 110. The second arm 420 may be coupled to the source assembly 110 by a source assembly coupling portion. The source assembly 110 may be rotatable about an axis parallel to the longitudinal direction of the second arm 420. In addition, the source assembly 110 may be rotatable about an axis extending in the left-right direction. Since the degree of freedom of movement of the source assembly 110 is high in this way, the user can adjust the radiation irradiation direction of the source assembly 110 to be perpendicular to the surface of the detector 120 by moving the source assembly 110.

FIG. 11 shows a plan view of the medical imaging device according to one embodiment of the present disclosure.

One end of the source assembly 110 may be coupled to one end of a source assembly bracket 1101. In addition, the other end of the source assembly 110 may be coupled to the other end of the source assembly bracket 1101. Here, one end may indicate the left side or right side, and the other end may indicate the right side or left side.

The source assembly bracket 1101 may have a right-angled C-shape. The source assembly bracket 1101 may include a bracket base 1211 extending in the left-right direction. In addition, the source assembly bracket 1101 may include a first bracket extension 1212 extending forward from a left side end of the bracket base 1211 and a second bracket extension 1213 extending forward from a right side end of the bracket base 1211. The source assembly 110 including the x-ray source and the collimator may be located between the first bracket extension 1212 and the second bracket extension 1213.

The second joint part 440 may include a first smart actuator 1231 coupled to one side of at least one of the first arm 410 and the second arm 420. Here, one side may indicate a left side surface. The first smart actuator 1231 may include the components described above with reference to FIG. 5.

The second joint part 440 may include a second smart actuator 1232 coupled to the other side of at least one of the first arm 410 and the second arm 420. Here, the other side may indicate a right side surface. The second smart actuator 1232 may include the same components as the first smart actuator 1231.

The first smart actuator 1231 and the second smart actuator 1232 may provide a driving force to an axis of rotation of the second arm 420 relative to the first arm 410. By the first smart actuator 1231 and the second smart actuator 1232 being provided on the second joint part in this way, an insufficient driving force of one smart actuator can be supplemented. In addition, when the smart actuator is provided on one of the right side and the left side of the second joint part 440, a problem may occur in durability because of the balance of the source arm tilting to one side due to the weight of the smart actuator, or there may be a problem that the medical imaging device 100 may sway while moving. Here, the problem in durability may mean that wear occurs on only one side. However, since the first smart actuator 1231 and the second smart actuator 1232 are provided at the left side and the right side, respectively, of the second joint part 440, the durability of the source arm increases because the left and right sides of the source arm are balanced, and the medical imaging device 100 can stably move in balance.

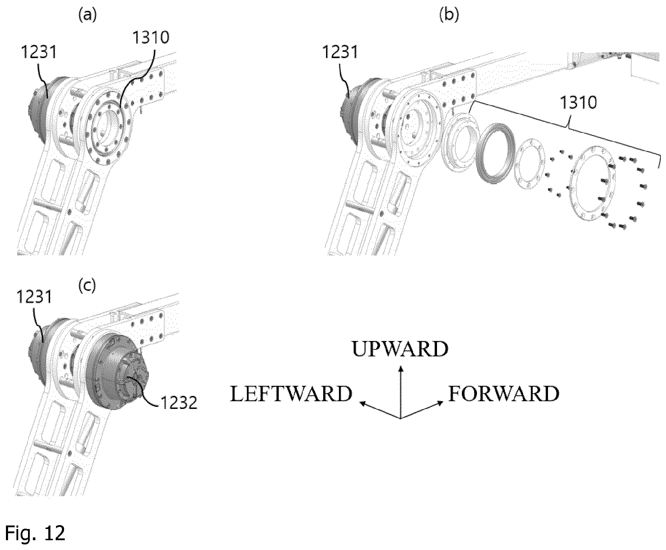

FIG. 12 is a view for describing the second joint part according to one embodiment of the present disclosure.

Referring to FIG. 12A, the second joint part 440 may include the first smart actuator 1231 and a swivel bearing 1310. The first smart actuator 1231 may include components identical to those shown in FIG. 5. The swivel bearing 1310 may be a component for making rotation of the second joint part 440 smooth. The swivel bearing 1310 may be mounted in place of the second smart actuator 1232 on the medical imaging device 100 as necessary. Since the first smart actuator 1231 is located on one side (left side), and the swivel bearing 1310 is located on the other side (right side), a phenomenon in which wear occurs only on one side of the components of the second joint part 440 can be mitigated.

FIG. 12B shows components included in the swivel bearing. Since the swivel bearing includes numerous components, there is a problem that assembly is difficult. In addition, when the assembly is performed incorrectly, the swivel bearing may not function properly, and a problem may occur.

FIG. 12C shows a case in which the second smart actuator 1232 is provided instead of the swivel bearing. That is, the second joint part 440 may include the first smart actuator 1231 and the second smart actuator 1232. Since the second smart actuator 1232 is modularized, assembly may be easy. In addition, by the first smart actuator 1231 and the second smart actuator 1232 being provided on the second joint part, an insufficient driving force of one smart actuator can be supplemented.

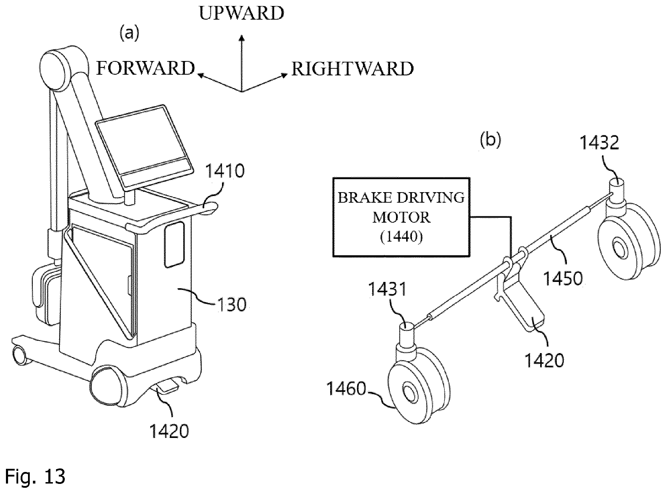

FIG. 13 is a view for describing a movement brake of a main body according to one embodiment of the present disclosure.

Referring to FIG. 13A, a brake pedal 1420 may be located on a lower end of the main body 130. When the brake pedal 1420 is lowered due to a user stepping on the brake pedal 1420, the medical imaging device 100 may be in an immovable state. The medical imaging device 100 should be fixed to capture a clear medical image and allow the user to irradiate only a desired site with radiation.

Conversely, when the brake pedal 1420 is lifted due to the user lifting the brake pedal 1420 with his or her foot, the medical imaging device 100 may be in a movable state. Since the mobile medical imaging device 100 moves to where a patient is present and captures a radiation image, user convenience may be high because there is no need to move a patient who is unwell. The user may move the medical imaging device 100 while holding a handle 1410 formed at the rear of the main body 130.

The medical imaging device 100 according to various embodiments of the present disclosure may include a brake driving motor 1440. The brake pedal 1420 of the medical imaging device 100 may move also due to the brake driving motor 1440. For example, the brake driving motor 1440 may rotate a brake driving shaft 1450 fixed to the brake pedal 1420. When the brake pedal 1420 is lowered due to the brake driving motor 1440 rotating the brake driving shaft 1450, the medical imaging device 100 may be in an immovable state. For example, the brake driving shaft 1450 may allow brake pads 1431 and 1432 to come into contact with portions of wheels 1460 to prevent rotation of the wheels 1460. When the brake pedal 1420 is lifted due to the brake driving motor 1440 rotating the brake driving shaft 1450 in the opposite direction, the medical imaging device 100 may be in a movable state. For example, the brake driving shaft 1450 may move the brake pads 1431 and 1432 to be distant from the wheels 1460 to not interfere with rotation of the wheels 1460. When the medical imaging device 100 has not received any input and has not moved for a predetermined amount of time, the controller 300 may automatically operate the brake driving motor 1440 to operate the brake. In addition, when the user pushes or pulls the medical imaging device 100 to move the medical imaging device 100, such a force may be detected, and the brake may be automatically released. The brake pedal 1420 may be manually moved by the user or automatically moved by the brake driving motor 1440. In this way, since the brake pedal 1420 is automatically controlled by the brake driving motor 1440, convenience of the medical imaging device 100 can be improved.

The present disclosure has been described above using various embodiments. Those of ordinary skill in the art to which the present invention pertains should understand that the present invention can be implemented in modified forms within the scope not departing from essential characteristics of the present invention. Therefore, the embodiments disclosed herein should be considered illustrative instead of limiting. The scope of the present invention is shown by the claims rather than the above description, and all differences within the scope equivalent to the claims should be construed as belonging to the present invention.

Meanwhile, the above-described embodiments of the present invention can be written by a computer executable program and may be implemented in a universal digital computer running the program using computer readable recording media. The computer readable recording media include storage media such as magnetic storage media (e.g., a read-only memory (ROM), a floppy disk, a hard disk, etc.) and optical readable media (e.g., a CD-ROM, a DVD, etc.).

## Claims

1. A medical imaging device comprising:
a main body that is able to travel;
a first arm coupled to the main body by a first joint part;
a second arm coupled to the first arm by a second joint part including a smart actuator; and
a controller for controlling the first joint part and the second joint part,
wherein the controller controls the first arm to be tilted at a predetermined fixed angle relative to the ground and fixed, controls the second joint part to rotate the second arm relative to the first arm based on at least one of a torque applied to the second joint part and an input of a user, and controls the second joint part so that a predetermined angle is formed between the first arm and the second arm based on an input of the user that is related to joint movement.

2. The medical imaging device of claim 1, wherein:
the controller measures a first torque applied to the second joint part;
the controller determines whether the first torque is higher than or equal to a predetermined threshold sensitivity torque of the second joint part;
when the first torque is higher than or equal to the threshold sensitivity torque of the second joint part, the controller controls the second joint part to rotate the second arm relative to the first arm; and
the threshold sensitivity torque of the second joint part is changeable.

3. The medical imaging device of claim 1, further comprising:
a source assembly that is coupled to the other end of the second arm and includes a second transceiver; and
a detector that generates a medical image by receiving radiation radiated from the source assembly and includes a first transceiver transmitting and receiving a signal to and from the second transceiver,
wherein, based on the first transceiver and the second transceiver, the controller controls the second joint part so that a radiation irradiation direction of the source assembly becomes perpendicular to a radiation reception surface of the detector.

4. The medical imaging device of claim 1, wherein:
the controller obtains a second torque caused by an external force while the second arm moves relative to the first arm due to driving of the second joint part;
the controller determines whether the second torque is higher than or equal to a predetermined threshold impact torque; and
when the second torque is higher than or equal to the predetermined threshold impact torque, the controller stops the driving of the second joint part.

5. The medical imaging device of claim 1, wherein the second arm includes:
a second-first arm that has one end coupled to the second joint part;
a second-second arm that has at least one portion inserted into a space formed inside the second-first arm and is able to move along the second-first arm; and
a telescopic arm driving part that is coupled to an inner portion of the second-first arm and provides a driving force for movement of the second-second arm relative to the second-first arm.

6. The medical imaging device of claim 1, wherein the second joint part includes:
a first smart actuator coupled to one side of at least one of the first arm and the second arm; and
a second smart actuator coupled to the other side of at least one of the first arm and the second arm,
wherein the first smart actuator and the second smart actuator provide a driving force to an axis of rotation of the second arm relative to the first arm.

7. A medical imaging device comprising:
a main body that is able to travel;
a first arm coupled to the main body by a first joint part;
a second arm coupled to the first arm by a second joint part and stretchable/contractible by a telescopic arm driving part; and
a controller for controlling the first joint part and the second joint part,
wherein the controller controls the first arm to be tilted at a predetermined fixed angle relative to the ground and fixed, controls the second joint part to rotate the second arm relative to the first arm based on at least one of a torque applied to the second joint part and an input of a user, and controls the second joint part so that a predetermined angle is formed between the first arm and the second arm based on an input of the user that is related to joint movement.

8. The medical imaging device of claim 7, wherein the second arm includes:
a second-first arm that has one end coupled to the second joint part;
a second-second arm that has at least one portion inserted into a space formed inside the second-first arm and is able to move along the second-first arm; and
a telescopic arm driving part that is coupled to an inner portion of the second-first arm and controls a driving force for movement of the second-second arm relative to the second-first arm.

9. The medical imaging device of claim 8, wherein the controller controls the second-second arm to move relative to the second-first arm based on one of a user input on a button related to a telescopic function and a force applied to the second-second arm by the user.

10. The medical imaging device of claim 8, wherein:
the controller obtains an outer force caused by an external force while the second-second arm moves relative to the second-first arm due to the telescopic arm driving part;
the controller determines whether the outer force is greater than or equal to a predetermined threshold impact force; and
when the outer force is greater than or equal to the predetermined threshold impact force, the controller stops the driving of the telescopic arm driving part.
